# FASCICULE DE BREVET EUROPEEN

(11) **EP 2 203 487 B1**
(45) Date de publication et mention de la délivrance du brevet: **11.06.2014**
(21) Numéro de dépôt: 08805103.2
(22) Date de dépôt: 07.10.2008
(51) Int. Cl.: C08F 30/04, B01D 15/00, B01J 31/22, C07C 229/14, C09K 11/06, H01F 1/42

(54) **PROCEDE DE PREPARATION DE MATERIAUX POLYMERIQUES DOPES PAR DES ELEMENTS METALLIQUES ET MATERIAUX OBTENUS PAR CE PROCEDE**
VERFAHREN ZUR HERSTELLUNG VON MIT METALLELEMENTEN DOTIERTEN POLYMERMATERIALIEN UND RESULTIERENDE MATERIALIEN
METHOD FOR PREPARING POLYMER MATERIALS DOPED WITH METAL ELEMENTS AND RESULTING MATERIALS

(30) Priorité: 08.10.2007 FR 0758126
(43) Date de publication de la demande: 07.07.2010
(73) Titulaire: Commissariat à l'Énergie Atomique et aux Énergies Alternatives, 75015 Paris (FR)
(72) Inventeur: BALLAND LONGEAU, Alexia, F-37000 Tours (FR); MOREAU, Louis, F-37350 Le Petit Pressigny (FR); THIBONNET, Jérôme, F-37250 Veigne (FR); VELASQUEZ, Emilie, F-03500 Meillard (FR)
(74) Mandataire: Ilgart, Jean-Christophe
(86) Numéro de dépôt international: PCT/EP2008/063389
(87) Numéro de publication internationale: WO 2009/047245

(56) Documents cités:
- EP-A2- 1 308 481
- WO-A2-2007/120999
- DATABASE CA [Online] CHEMICAL ABSTRACTS SERVICE, COLUMBUS, OHIO, US; 2002, BAXTER, ELLEN W. ET AL: "Reductive aminations of carbonyl compounds with borohydride and borane reducing agents" XP002517937 extrait de STN Database accession no. 2008:1383655
- KAWAMURA, HIROAKI ET AL: "Cu(II)-assisted helicity induction on a poly(phenylacetylene) derivative bearing an achiral glycine residue with amino acids in water" CHEMISTRY LETTERS , 32(11), 1086-1087 CODEN: CMLTAG; ISSN: 0366-7022, 2003, XP009113218

## Description

### DOMAINE TECHNIQUE

La présente invention a trait à un procédé de préparation de matériaux polymériques dopés par des éléments métalliques.

Ces matériaux polymériques trouvent leur application dans le champ global des applications propres aux matériaux polymériques dopés avec des éléments métalliques, telles que la catalyse supportée, les matériaux luminescents, les matériaux magnétiques, les matériaux à empreinte ionique. En particulier, ils trouvent leur application dans l'élaboration de cibles laser utilisées lors d'expériences de fusion par confinement inertiel.

### ÉTAT DE LA TECHNIQUE ANTÉRIEURE

Eu égard au champ d'application extrêmement vaste de ce type de matériaux, de nombreuses équipes ont axé leur recherche sur des procédés d'élaboration de tels matériaux.

Une première stratégie a consisté à imprégner des matériaux polymériques par des solutions de sel métallique.

Ainsi, Rinde et al. dans US 4,261,937 décrit une méthode de préparation de mousses polymériques dopées par un élément métallique consistant à verser un gel polymérique dans une solution aqueuse comprenant un sel dudit élément métallique. Le gel est ensuite mis en présence d'une série de solvants de polarité décroissante, afin d'éliminer l'eau introduite. Chaque solvant utilisé doit être capable de solubiliser le solvant précédent et est saturé par le sel métallique choisi.

Cette méthode présente toutefois l'inconvénient majeur que la répartition de l'élément métallique ne peut pas être parfaitement homogène au niveau atomique, car il se produit des phénomènes de cristallisation de sels métalliques au séchage, qui s'ensuit par la formation de nano- ou microcristaux dans le matériau. D'autre part, du fait que l'imprégnation est réalisée sur un gel polymérique, la diffusion des éléments métalliques ne se produit pas dans la totalité du gel.

Une deuxième stratégie a consisté, non pas à imprégner des matériaux polymériques par une solution de sel métallique, mais à doper ce type de matériaux par l'utilisation de particules métalliques solides.

Ainsi, Faith et al. dans Fusion Science and Technology, vol.45, Mar. 2004, p.90-94, décrivent un procédé de réalisation de mousses chargées de particules solides d'or, ces mousses étant réalisées par polymérisation *in situ* en présence desdites particules du monomère triméthylolpropanetriacrylate. Toutefois, ce procédé ne permet pas une répartition homogène au sein du matériau, le dopant étant sous la forme de particules agglomérées comprenant plusieurs dizaines voire centaines d'atomes.

Enfin, une stratégie plus récente a consisté à réaliser des matériaux polymériques par copolymérisation de monomères organométalliques, c'est-à-dire de monomères dont l'élément métallique est lié de façon covalente à un ou plusieurs atomes du monomère (tel que décrit dans Tetrahedron Letters, 2000, 41, 4905).

Toutefois, ce type de stratégie peut s'avérer difficile à mettre en oeuvre, car il nécessite le développement d'une chimie spécifique à chaque métal susceptible d'être utilisé comme élément métallique dopant.

Ainsi, il existe un véritable besoin pour un procédé de préparation de matériaux polymériques dopés par un élément métallique, qui permette une incorporation homogène dudit élément métallique dans le matériau et qui ne nécessite pas le développement d'une chimie spécifique à l'élément métallique à incorporer.

### EXPOSÉ DE L'INVENTION

Pour ce faire, les Inventeurs ont mis en place, de façon judicieuse, un procédé de préparation d'un matériau polymérique dopé par un élément métallique alliant à la fois la technologie de la polymérisation et la technologie de la chimie de coordination.

Ainsi, l'invention a trait, de façon générale, à un procédé de préparation d'un matériau polymérique dopé par au moins un élément métallique comprenant une étape de polymérisation d'un complexe de coordination dudit élément métallique formé dudit élément et d'un ou plusieurs ligands dudit élément, le ou lesdits ligands appartenant à au moins un monomère comprenant au moins un groupe éthylénique.

On précise que, par complexe de coordination, on entend, au sens de l'invention, un édifice polyatomique comprenant l'élément métallique dopant autour duquel des groupes appartenant à au moins un monomère sont liés par des liaisons de coordination, la liaison de coordination étant créée par apport d'un doublet d'électrons appartenant auxdits groupes dans une orbitale vide de l'élément métallique.

Le procédé de l'invention présente ainsi les avantages suivants :
- il permet l'incorporation, dans des matériaux polymériques, d'une large diversité d'éléments métalliques, du fait que la liaison entre les éléments métalliques et le ou les monomères s'effectue par liaison de coordination ;
- il permet une répartition de l'élément métallique à l'échelle atomique ;
- il permet une incorporation de taux élevés d'élément métallique, ledit taux étant fonction de la quantité de complexe de coordination mis en jeu lors de l'étape de polymérisation.

Selon l'invention, les monomères comportant des groupes susceptibles de constituer des ligands sont des monomères comportant au moins un groupe porteur d'un doublet libre, en particulier un groupe amine, et éventuellement au moins un groupe chargé négativement, en particulier un groupe carboxylate. Des monomères avantageux peuvent comprendre à la fois au moins un groupe amine et au moins un groupe carboxylate, ces deux types de groupes pouvant provenir d'un reste d'acide aminé.

Plus précisément, des monomères susceptibles d'être utilisés dans le procédé de l'invention et de former des complexes de coordination peuvent répondre à la formule (I) suivante : 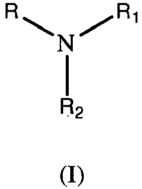 dans laquelle :
- R représente un groupe choisi parmi les groupes de formules suivantes : 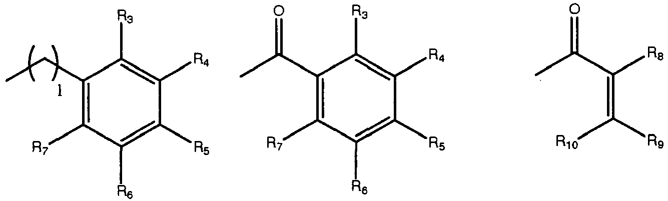
- R₁ et R₂ représentent, indépendamment, H, un groupe alkyle, un groupe aryle, un groupe de formules suivantes : 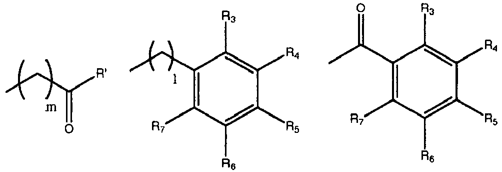 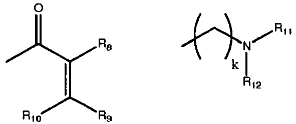 R₁₁ et R₁₂ correspondant, indépendamment, à des groupes répondant à la même définition que R₁ et R₂ donnée ci-dessus ;
- R' est un groupe OR₁₃, amine;
- R₃, R₄, R₅, R₆, R₇, représentent, indépendamment, H, un groupe éthylénique, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, -O-alkyle étant éventuellement perfluorés, un ou plusieurs atomes d'oxygène, d'azote, de soufre et/ou de sélénium pouvant s'intercaler dans lesdits groupes, à condition que l'un au moins des R₃, R₄, R₅, R₆, R₇ représente un groupe éthylénique;
- R₈, R₉ et R₁₀ représentent, indépendamment, H, un groupe éthylénique, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, -O-alkyle étant éventuellement perfluorés, un ou plusieurs atomes d'oxygène, d'azote, de soufre et/ou de sélénium pouvant s'intercaler dans lesdits groupes ;
- R₁₃ représente H, un métal, tel qu'un métal alcalin, un groupe alkyle, un groupe aryle, un groupe acyle, un groupe alkylaryle, lesdits groupes alkyle, aryle, alkylaryle étant éventuellement perfluorés et un ou plusieurs atomes d'oxygène, de soufre et/ou de sélénium pouvant s'intercaler dans lesdits groupes ;
- k, 1 et m sont des entiers allant de 0 à 20 ;
et les sels de ceux-ci.

Avant d'entrer plus en détail dans la description des monomères susmentionnés, nous proposons les définitions suivantes.

Par groupe alkyle, on entend, généralement, dans ce qui précède et ce qui suit, un groupe alkyle linéaire ou ramifié comprenant de 1 à 20 atomes de carbone ou cyclique comprenant de 3 à 20 atomes de carbone. On peut citer, à titre d'exemples, le groupe méthyle, éthyle, n-propyle, i-propyle, n-butyle, n-dodécanyle, i-butyle, t-butyle, cyclopropyle, cyclohexyle.

Par groupe aryle, on entend, généralement, dans ce qui précède et ce qui suit, un groupe aryle comprenant de 6 à 20 atomes de carbone. On peut citer, à titre d'exemples, le groupe benzyle, naphtyle, biphényle.

Par groupe alkylaryle, on entend, généralement, dans ce qui précède et ce qui suit, un groupe aryle de même définition que celle donnée ci-dessus, ledit groupe étant substitué par au moins un groupe alkyle de définition identique à celle donnée ci-dessus.

Par groupe -O-alkyle, -O-aryle, on entend un groupe alkyle ou un groupe aryle, répondant à la même définition que celle donnée ci-dessus, le groupe alkyle ou aryle étant, dans ce cas, relié à une autre partie du monomère par l'intermédiaire d'un atome d'oxygène.

Par groupe perfluoré, on entend un groupe dont tous les atomes d'hydrogène sont substitués par des atomes de fluor.

Lorsque l'on précise que un ou plusieurs atomes d'oxygène, d'azote, de soufre et/ou de sélénium pouvant s'intercaler dans lesdits groupes (à savoir les groupes alkyle, aryle, alkylaryle, -O-aryle, -O-alkyle), cela signifie, en d'autres termes, qu'un atome de carbone est remplacé par un groupe -O-, -S-, -N- ou -Se-.

Par groupe éthylénique, on entend un groupe carboné comprenant deux atomes de carbone liés par une double liaison, ce groupe étant susceptible d'être polymérisé par voie radicalaire. Un groupe éthylénique particulier est un groupe vinyle CH₂=CH-, (alkyl)acrylate, tel qu'un groupe (méth)acrylate.

Par groupe acyle, on entend un groupe -CO-alkyle, le groupe alkyle répondant à la même définition que celle donnée ci-dessus.

Par sel, on entend les composés de structure ionique. Par exemple, on peut citer les sels carboxylates métalliques, lorsque R' correspond à OR₁₃ avec R₁₃ étant un métal. Dans ce cas de figure, par métal, on entend classiquement un métal monovalent, tel qu'un métal alcalin, comme Na, K.

Par élément métallique, on entend, classiquement, un métal alcalin, un métal alcalino-terreux, un métal de transition, un lanthanide, un actinide ainsi que les éléments Al, Ga, Ge, In, Sn, Sb, Tl, Pb, Bi et Po.

En particulier, l'élément métallique est avantageusement un lanthanide, tel que l'ytterbium.

On précise que les indices k, l, m représente le nombre de répétitions du motif pris entre parenthèses, ce nombre pouvant aller de 0 à 20.

Des monomères particuliers peuvent être ceux, pour lesquels R est un groupe de formule : 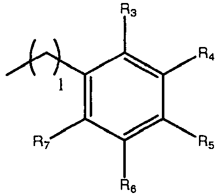 et l'un au moins des R¹ et R² est un groupe de formule : 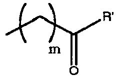 R₃ à R₇, R', l et m ayant les mêmes significations que celles explicitées ci-dessus, avec toujours la condition que l'un au moins des R₃ à R₇ représente un groupe éthylénique.

Plus particulièrement, des monomères conformes à la définition donnée ci-dessus sont des monomères pour lesquels R est un groupe de formule : 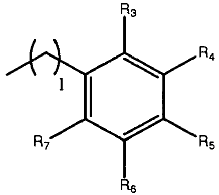 R₁ est un groupe de formule : 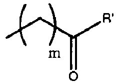 et R₂ est un atome d'hydrogène, l et m, R₃ à R₇ et R' ayant les mêmes significations que celles données ci-dessus, avec la condition que l'un au moins des R₃ à R₇ représente un groupe éthylénique. En particulier, 1 et m peuvent être égaux à 1.

Un monomère particulier de ce type répond à la formule (II) suivante : 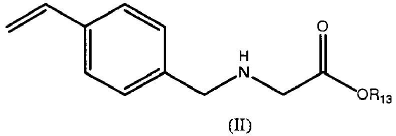 R₁₃ représentant, en particulier, H, un métal tel qu'un métal alcalin (comme Na, K) ou un groupe alkyle, tel qu'un groupe éthyle.

Un autre groupe de monomères tombant sous le coup de la définition des monomères de formule (I) correspond aux monomères pour lesquels R est un groupe de formule : 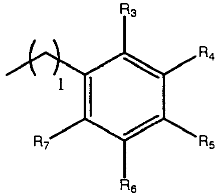 R₁ est un groupe de formule : 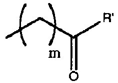 et R₂ est un groupe de formule : 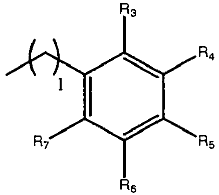 l et m, R₃ à R₇ et R' ayant les mêmes significations que celles données ci-dessus, avec la condition que l'un au moins des R₃ à R₇ représentent un groupe éthylénique. En particulier, l et m peuvent être égaux à 1.

Un monomère particulier de ce type répond à la formule (III) suivante :

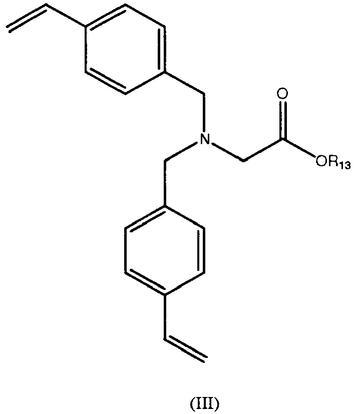

R₁₃ représentant, en particulier, H, un métal (tel qu'un métal alcalin comme Na, K) ou un groupe alkyle, tel qu'un groupe éthyle.

D'autres monomères susceptibles d'être utilisés, avantageusement, dans le procédé de l'invention peuvent être des monomères comportant une amine cyclique comprenant au moins deux atomes d'azote.

Des monomères particuliers de ce type répondent à la formule (IV) suivante : 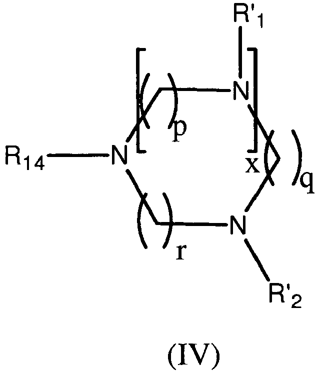 dans laquelle :
- R₁₄ représente un groupe choisi parmi les groupes de formules suivantes : 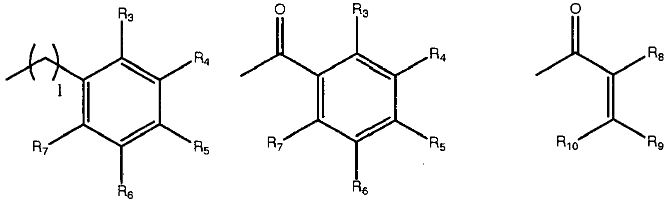 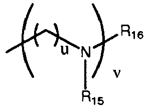
- R'₁ et R'₂ représentent, indépendamment, un groupe alkyle, un groupe aryle, un groupe de formules suivantes : 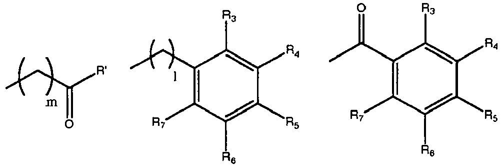 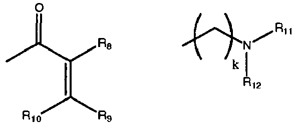 R₁₁ et R₁₂ correspondant, indépendamment, à des groupes répondant à la même définition que R'₁ et R'₂ donnée ci-dessus ;
- R' est un groupe OR₁₃, amine ;
- R₃, R₄, R₅, R₆ et R₇ représentent, indépendamment, H, un groupe éthylénique, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle,
- O-aryle, -O-alkyle étant éventuellement perfluorés, un ou plusieurs atomes d'oxygène, d'azote, de soufre et/ou de sélénium pouvant s'intercaler dans lesdits groupes, à condition que l'un au moins des R₃, R₄, R₅, R₆, R₇ représente un groupe éthylénique;

- R₈, R₉ et R₁₀ représentent, indépendamment, H, un groupe éthylénique, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, - O-alkyle étant éventuellement perfluorés, un ou plusieurs atomes d'oxygène, d'azote, de soufre et/ou de sélénium pouvant s'intercaler dans lesdits groupes ;
- R₁₅ représente un groupe de formules suivantes : 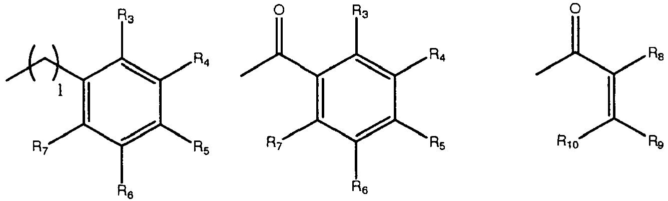 les R₃ à R₁₀ étant tels que définis ci-dessus ;
- R₁₃ représente H, un métal, un groupe alkyle, un groupe aryle, un groupe acyle, un groupe alkylaryle, lesdits groupes alkyle, aryle, alkylaryle étant éventuellement perfluorés et un ou plusieurs atomes d'oxygène, de soufre et/ou de sélénium pouvant s'intercaler dans lesdits groupes ;
- R₁₆ représente un groupe de formule : 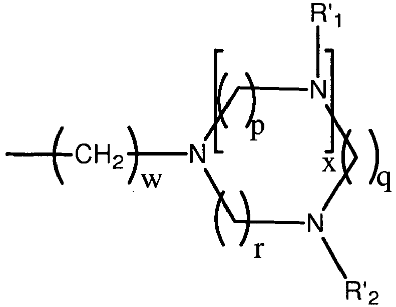 les R'₁ et R'₂ étant tels que définis ci-dessus ;
- k, l, m, u, p, q, r, x et w sont des entiers allant de 0 à 20, v est un entier allant de 1 à 20, à la condition que, lorsque x est égal à 0, (r+q) est au moins égale à 2, et lorsque x est égal à 1, au moins l'un des p, q, r est différent de 0.

On précise que p, q, r et x correspondent au nombre de répétitions du motif pris entre parenthèses (pour p,q et r) ou entre crochets (pour x).

Avantageusement, des monomères intéressants sont des monomères, pour lesquels R₁₄ représente un groupe de formule suivante : 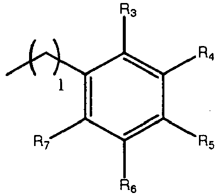 et l'un au moins des R'₁ et R'₂ représente 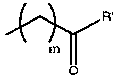 les R₃ à R₇, R', l et m répondant à la même définition que celle donnée ci-dessus, p, q, r et x étant avantageusement, au moins égaux à 1, à la condition toujours que l'un au moins des R₃ à R₇ représente un groupe éthylénique.

Plus particulièrement, des monomères conformes à la définition donnée ci-dessus sont des monomères pour lesquels R₁₄ est un groupe de formule : 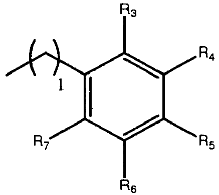 R'₁ et R'₂ représentent un groupe de formule : 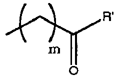 les R₃ à R₇, R', l et m répondant à la même définition que celle donnée ci-dessus, p, q, r et x étant, avantageusement au moins égaux à 1, à la condition toujours que l'un au moins des R₃ à R₇ représente un groupe éthylénique. En particulier, l et m peuvent être des entiers égaux à 1 et p, q, r et x peuvent être des entiers égaux à 2.

Un monomère particulier répondant à la définition ci-dessus est un monomère répondant à la formule (V) suivante : 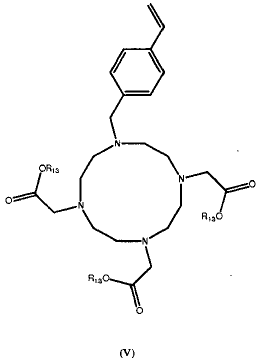 R₁₃ représentant, en particulier, H, un groupe alkyle, tel qu'un groupe éthyle, un métal.

Ainsi, pour le monomère de formule (V), R₁₄ correspond à un groupe de formule : 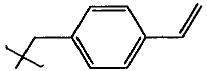 tandis que R₁ et R₂ correspondent à un groupe de formule -CH₂-COOR₁₃, et p, q, r et x sont des entiers égaux à 2.

Avantageusement, R₁₄ peut représenter, également, un groupe de formule : 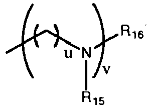 et l'un au moins des R'₁ et R'₂ représente : 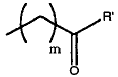 R₁₅, R₁₆, R', m, u et v répondant aux mêmes définitions que celles données ci-dessus, et p, q, r et x étant, avantageusement, au moins égaux à 1.

On précise que, lorsque R₁₄ correspond à un groupe de formule : 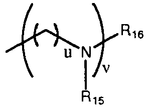 les monomères peuvent être représentés par la formule générale suivante :

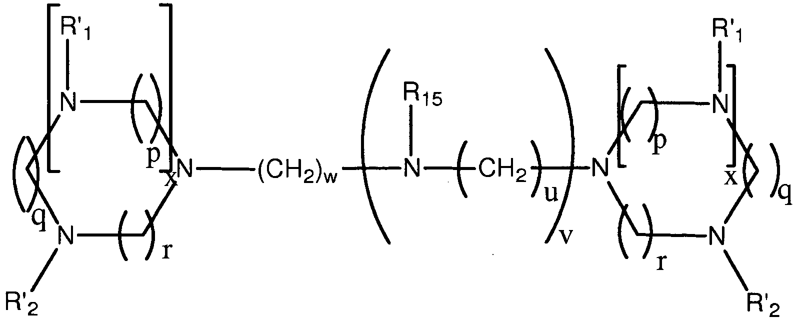

Un groupe de monomères particuliers tombant sous le coup de la définition donnée ci-dessus correspond à ceux pour lesquels R₁₅ correspond à un groupe de formule : 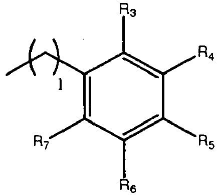 l, et les R₃ à R₇ répondant à la même définition que celle donnée ci-dessus, avec la condition que l'un au moins des R₃ à R₇ représentent un groupe éthylénique, et les groupes R'₁ et R'₂ représentent un groupe de formule : 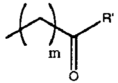 avec m et R' répondant aux mêmes définitions que celles données ci-dessus, p, q, r et x étant, avantageusement, au moins égaux à 1. En particulier, p, q, r, x, u, v et w représentent, par exemple, un entier égal à 2.

Un monomère particulier entrant sous le coup de la définition précédente répond à la formule (VI) suivante : 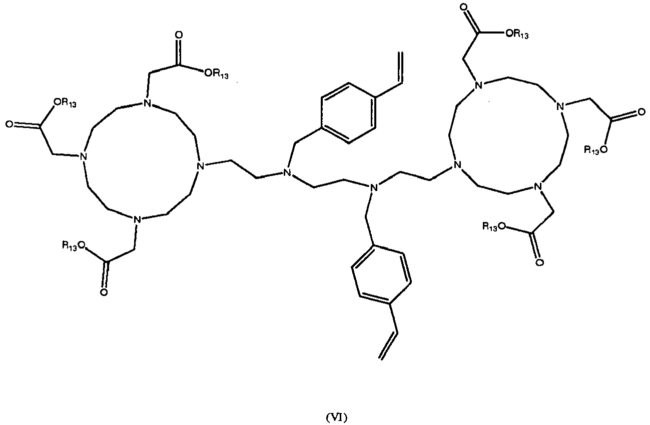 R₁₃ représentant, en particulier, H, un métal, ou un groupe alkyle, tel qu'un groupe éthyle,
et les sels éventuels de celui-ci.

Ainsi, pour ce monomère de formule (VI), les R₁₅ représentent un groupe de formule : 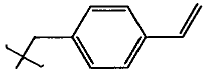 tandis que R'₁ et R'₂ correspondent à un groupe de formule -CH₂-COOR₁₃, et p, q, r, x, u, v et w sont des entiers égaux à 2.

De part la présence d'atomes d'azote porteurs de doublets libres, ces monomères sont aptes à former un complexe de coordination avec l'élément métallique approprié.

L'élément métallique peut être un métal alcalin, un métal alcalino-terreux, un métal de transition, un lanthanide, un actinide ainsi que les éléments Al, Ga, Ge, In, Sn, Sb, Tl, Pb, Bi et Po.

En particulier, l'élément métallique est avantageusement un lanthanide, tel que l'ytterbium.

La formation de complexes de coordination sera explicitée davantage ci-dessous.

Ainsi, avant l'étape de polymérisation, le procédé de l'invention peut comprendre une étape de préparation du complexe de coordination susmentionné. Par exemple, la formation du complexe peut consister à mettre en contact les monomères susmentionnés avec une solution aqueuse et/ou organique comprenant l'élément métallique approprié.

Classiquement, l'étape de polymérisation du procédé de l'invention se déroule, outre la présence du complexe de coordination, en présence éventuellement d'un initiateur de polymérisation et éventuellement d'un solvant porogène et d'un ou plusieurs comonomères.

Avantageusement, lorsque l'étape de polymérisation se déroule sans comonomères, les monomères entrant dans la constitution des complexes de coordination comprendront au moins deux groupes éthyléniques.

L'initiateur de polymérisation est un amorceur radicalaire classiquement choisi parmi les composés peroxydes, azonitriles (tel que le 2,2'-azobisisobutyronitrile), azoesters, azoamides.

L'initiateur peut être introduit, dans le milieu de polymérisation, selon des quantités variables, par exemple, selon des quantités pouvant aller de 0 à 50% massique, par rapport à la masse totale de monomères mis en jeu.

Le solvant porogène peut être un solvant organique polaire, apolaire et peut être choisi parmi les solvants éthers (tels que le tétrahydrofurane), le diméthylsulfoxyde, des solvants phthalates (tels que le diméthylphthalate, le dibutylphthalate), des solvants alcooliques (tels que le méthanol, l'éthanol), des solvants aromatiques (tels que le toluène, le fluorobenzène), des solvants cétones.

L'étape de polymérisation peut être réalisée en présence d'un ou plusieurs comonomères, lesdits comonomères étant, généralement différent des monomères entrant dans la constitution des complexes de coordination.

Ces comonomères peuvent être choisis parmi les monomères styréniques ou les monomères acrylates.

Avantageusement, les comonomères comprennent au moins deux groupes éthyléniques, assurant ainsi un rôle d'agent de réticulation. Les matériaux ainsi obtenus présentent une bonne tenue mécanique.

Des comonomères susceptibles d'être utilisés peuvent être des monomères styréniques de formule (VII) suivante : 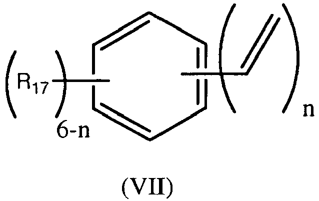 dans laquelle les (6-n) R₁₇, identiques ou différents, représentent un atome d'hydrogène, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, -O-alkyle étant éventuellement perfluorés et n est un entier allant de 1 à 3, de préférence, n étant égal à 2.

En particulier, un comonomère approprié peut être le divinylbenzène, en particulier le 1,4-divinylbenzène.

Des comonomères susceptibles d'être utilisés peuvent être également des composés acrylates de formule (VIII) suivante : 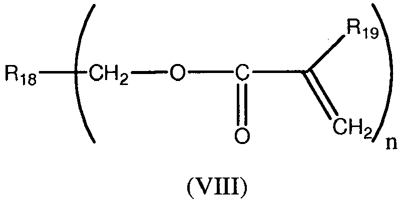 dans laquelle R₁₈ représente un groupe alkyle, R₁₉ représente H ou un groupe alkyle et n étant un entier allant de 1 à 3.

En particulier, un comonomère approprié de ce type peut être le triméthylolpropanetriacrylate (connu sous l'abréviation TMPTA) de formule suivante :

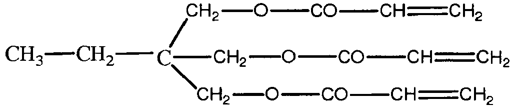

Classiquement, l'étape de polymérisation est réalisée à une température allant de 40 à 100°C.

En particulier, l'étape de polymérisation peut consister en :
- la copolymérisation d'un complexe de coordination formé d'un monomère de formule (V) et d'ytterbium, en présence de divinylbenzène ;
- la polymérisation d'un complexe de coordination formé de monomères de formule (II) et d'ytterbium ;
- la copolymérisation d'un complexe de coordination formé de monomères de formule (II) et d'ytterbium, en présence de divinylbenzène ou de triméthylolpropanetriacrylate ;
- la polymérisation d'un complexe de coordination formé de monomères de formule (III) et de cuivre.

Après l'étape de polymérisation, un gel est obtenu, correspondant à un réseau tridimensionnel, dont la structure est imprégnée par le solvant. Le gel, une fois synthétisé, doit être séché, afin d'obtenir le matériau polymérique dopé sec.

Ainsi, le procédé comprend avantageusement une étape de séchage du gel obtenu, cette étape étant avantageusement une étape de séchage supercritique au CO₂. Pour ce faire, cette étape de séchage supercritique au CO₂ peut être précédée d'une étape d'échange de solvant consistant à remplacer le solvant présent dans les pores du gel par un solvant miscible au CO₂. Cette étape de séchage supercritique au CO₂ permet notamment de respecter l'intégrité physique de la mousse.

L'on obtient, grâce au procédé de l'invention, des matériaux polymériques dopés par un élément métallique, présentant un pourcentage élevé d'élément métallique (pouvant être supérieur à 20% massique) et avec une répartition à l'échelle moléculaire de l'élément métallique au sein du matériau.

Ces matériaux peuvent être utilisés dans de nombreux domaines nécessitant la mise en oeuvre de matériaux dopés par des éléments métalliques et notamment dans l'élaboration d'éléments de cibles laser utilisés, en particulier, dans des expériences de fusion par confinement inertiel.

Des matériaux particuliers conformes à l'invention sont des matériaux obtenus par :
- la copolymérisation d'un complexe de coordination formé d'un monomère de formule (V) et d'ytterbium, en présence de divinylbenzène ;
- la polymérisation d'un complexe de coordination formé de monomères de formule (II) et d'ytterbium ;
- la copolymérisation d'un complexe de coordination formé de monomères de formule (II) et d'ytterbium, en présence de divinylbenzène ou de triméthylolpropanetriacrylate ;
- la polymérisation d'un complexe de coordination formé de monomères de formule (III) et de cuivre.

Ils peuvent être utilisés en tant que catalyseur, en tant que matériaux luminescents, magnétiques.

Enfin, ils peuvent être utilisés en tant que matériaux à empreinte ionique. Pour ce faire, les matériaux dopés obtenus par le procédé de l'invention peuvent être soumis à un traitement acide, destiné à éliminer une partie des éléments métalliques complexés dans ledit matériau. Les sites vacants constituent ainsi des empreintes spécifiques de l'élément spécifique du métal initialement introduit. De ce traitement, il en résulte un matériau dit « à empreinte ionique », capable de piéger sélectivement l'élément métallique « imprimé » lors d'une mise en contact avec un fluide comprenant ledit élément métallique. Ce type de matériaux peut ainsi être utilisé pour l'extraction sélective de métaux, notamment, lors du retraitement d'effluents de combustibles nucléaires, tel que la séparation des lanthanides, ou encore la décontamination de fluides biologiques.

Parmi les monomères susceptibles d'être utilisés dans le cadre du procédé de l'invention, certains sont nouveaux.

Ainsi, l'invention a trait également à de nouveaux monomères, ces monomères correspondant à ceux de formules générales (I) et (IV) définies ci-dessus et à ceux des formules particulières (II), (III), (V) et (VI) telles que définies ci-dessus.

Ces monomères sont fabriqués classiquement par des réactions de substitutions nucléophiles entre des amines et des composés halogénés.

Ainsi, à titre d'exemple, la préparation du monomère final de formule (V), avec R₁₃ correspondant à H ou Na, peut se dérouler, par exemple selon le schéma réactionnel suivant en trois étapes :
- une première étape 1) consistant en une fonctionnalisation avec un composé bromoacétate d'alkyle ;
- une seconde étape 2) consistant en une alkylation ;
- une troisième étape 3) consistant en une déprotection en milieu acide ou basique pour libérer les groupes carboxyliques

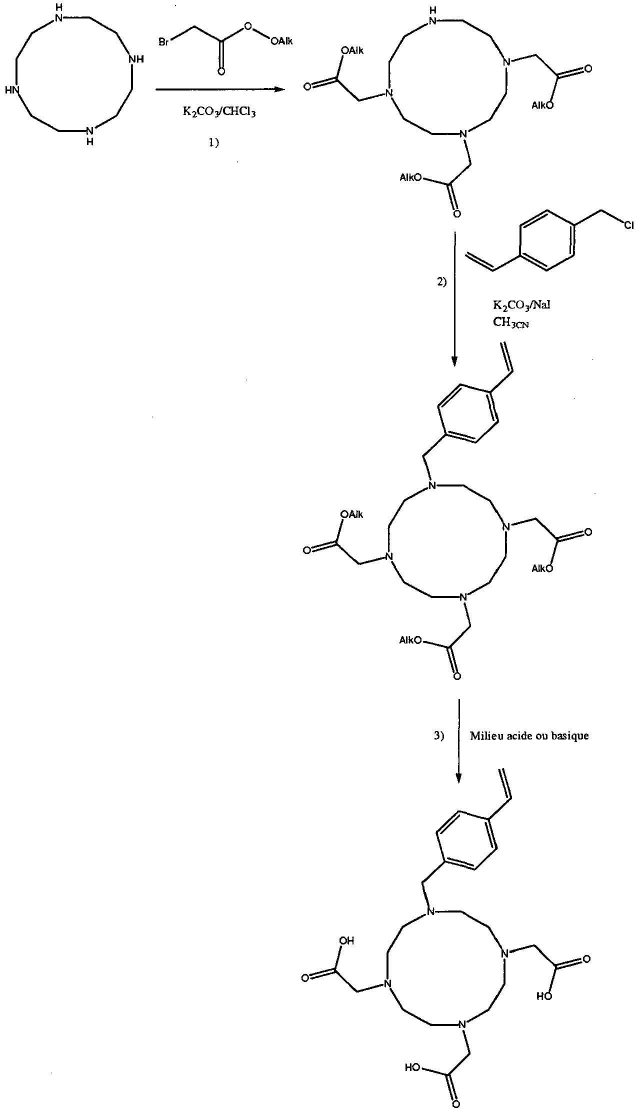

Alk correspondant à un groupe alkyle, tel qu'un groupe méthyle, éthyle, propyle, butyle (en particulier, t-butyle).

Comme mentionné précédemment, les monomères tels que définis ci-dessus sont utilisés sous forme de complexe de coordination avec au moins un élément métallique dans le procédé de préparation des matériaux polymériques de l'invention.

L'élément métallique peut être un métal alcalin, un métal alcalino-terreux, un métal de transition, un lanthanide, un actinide ainsi que les éléments Al, Ga, Ge, In, Sn, Sb, Tl, Pb, Bi et Po.

En particulier, l'élément métallique est avantageusement un lanthanide, tel que l'ytterbium.

Les complexes de coordination susmentionnés sont obtenus par mise en contact des monomères de formules (I) à (VI) telles que définies ci-dessus, éventuellement sous forme de sels, avec un composé de MXₙ, M représentant l'élément métallique, X un anion et n la valence dudit élément métallique.

A titre d'exemples, X peut être un chlorure, bromure, fluorure, iodure, iodate, nitrate, sulfate, sulfonate, sulfite, nitrate, nitrite, phosphate, phosphite, cyanure, azidure, hydroxyle, chlorate, perchlorate, acétate, trifluorométhanesulfonate (ou triflate), trifluoroacétate, trichloroacétate, alcoxyde, acétylacétonate, cyclopentadiényle, alcynure.

La réaction de formation des complexes peut être effectuée en milieu aqueux, organique ou en émulsion multiphasique.

Des complexes particuliers sont des complexes de monomères de formule (II), (III) ou (V) avec un élément métallique, tel que l'ytterbium ou le cuivre.

L'invention va maintenant être décrite plus en détail par rapport aux exemples donnés ci-dessus, fournis à titre illustratif et non limitatif.

### EXPOSÉ DÉTAILLÉ DE MODES DE RÉALISATION PARTICULIERS

### EXEMPLE 1

Cet exemple illustre la préparation d'un monomère de formule suivante : 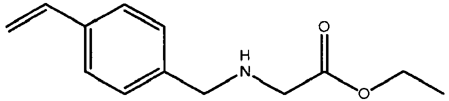 dit composé Gly²OEt.

Dans un ballon bicol de 250 mL surmonté d'un réfrigérant, sous atmosphère d'argon, on charge 21,9 g d'hydrochlorure de glycinate d'éthyle (3 éq., 157,2 mmoles), 18,1 g de carbonate de potassium (2,5 éq., 131,0 mmoles) et 150 mL d'acétonitrile anhydre. Le mélange est agité vigoureusement durant 10 minutes à température ambiante et on additionne 8 g de chlorure de 4-vinylbenzène (1 éq., 52,4 mmoles). Le milieu réactionnel est porté à 70°C et agité vigoureusement durant 18 heures. Le milieu réactionnel est ensuite laissé revenir à température ambiante et filtré afin d'éliminer les sels. Après évaporation du filtrat sous pression réduite, on obtient 19,4 g de produit brut sous la forme d'une huile visqueuse de couleur jaune. Le produit brut est purifié sur gel de silice neutralisé par de la triéthylamine (300 g de silice, éluant : 100% dichlorométhane puis mélange dichlorométhane/méthanol 98/2). On isole 11,3 g de produit sous la forme d'un liquide jaune pâle. Rendement : 98%.

### Caractéristiques physico-chimiques:

**RMN¹H** (200, 13 MHz, CDCl₃) : δ_{H} 1,27 (3H, t, *j* = 7,2 Hz, CH₂*CH*₃), 2,00 (1H, s, NH), 3,40 (2H, s, *-CH₂CO-),* 4,13 (2H, s, *CH₂Ar.),* 4,18 (2H, q,j = 7,2/7,0 Hz, *CH₂CH₃),* 5,21 (1H, dd,j = 0,7/10,8 Hz, CH=*CH₂*), 5,74 (1H, *d, j =* 0,7/17,6 Hz, CH=*CH₂*), 6,70 (1H, dd,j = 10, 8/17,6 Hz, *CH=*CH₂.), 7,28 (2H, d,*j* = 8,0 Hz, *CH-Ar),* 7,37 (2H, d, *j* = 8,2 Hz, *CH-Ar)*
**RMN*¹³*C** (50,32 MHz, CDCl₃) : δ_{c} 13,8; 43,6; 52,5; 60,3; 113,0; 125,9 (2C); 128,0 (2C); 136,1; 136,2; 139,0; 173,9

### EXEMPLE 2

Cet exemple illustre la préparation du monomère de formule suivante : 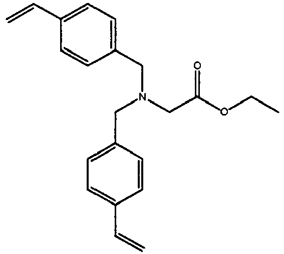 dit composé Gly^{2'} OEt.

Dans un ballon de 100 mL surmonté d'un réfrigérant, sous atmosphère d'argon, on charge 1 g d'hydrochlorure de glycinate d'éthyle (7,1 mmoles), 3,37 g de carbonate de potassium (1,7 éq., 12,2 mmoles), 2,18 g de chlorure de 4-vinylbenzyle (2 éq., 14,3 mmoles), 5% molaire d'iodure de sodium et 30 mL d'acétonitrile anhydre. Le mélange est porté à 70°C et agité durant 16 heures. Le milieu réactionnel est ensuite laissé revenir à température ambiante et filtré afin d'éliminer les sels. Le solvant est évaporé sous pression réduite et le résidu est repris dans 20 mL déther éthylique. Les sels, qui ont à nouveau précipités, sont éliminés par filtration. Après évaporation du filtrat sous pression réduite, on obtient 2,17 g d'une huile de couleur jaune pure à 95%. Rendement : 87%.

### Caractéristiques physico-chimiques:

**RMN¹H** (200,13 MHz, CDCl₃) : δ_{H} 1,29 (3H, t,*j* = 8,0 Hz, CH₂*CH₃*), 3,32 (2H, s, CH₂CO), (3,83 4H, s, *CH₂Ar),* 4,19 (2H, q,j = 8,0/6,0 Hz, *CH₂*CH₃*), 5, 24* (2H, dd, *j* = 0,8/12,0 Hz, CH=*CH₂*), 5,77 (2H, dd, *j* = 0,8/18,0 Hz, CH*=CH₂),* 6,75 (2H, dd, *j* = 10, 8/17,6 Hz, *CH=CH₂),* 7,0 - 7,40 (8H, m, *CH-Ar)*

### EXEMPLE 3

Cet exemple illustre la préparation du monomère de formule suivante :

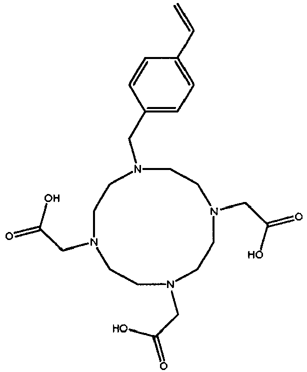

### a) 1^{ère} étape : Synthèse du 1,4,7-Tris(tert-butoxycarboxyméthyle)-1,4,7,10-tétraazacyclododécane de formule suivante :

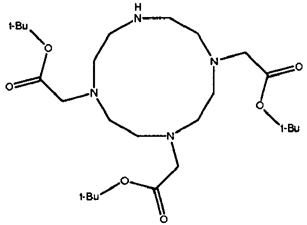

Dans un ballon surmonté d'une ampoule à brome, on place 1 g de cyclène (5,8 mmoles), 0,8 g de carbonate de potassium (5,8 mmoles, 1 éq.) et 150 mL de chloroforme anhydre. Le mélange est vigoureusement agité et on additionne goutte à goutte, durant 4 heures, une solution de tert-butylbromoacétate (3 éq., 3,39 g, 17,4 mmoles) dans 50 mL de chloroforme anhydre. Après addition complète, le milieu est agité à température ambiante durant 72 heures. Le milieu réactionnel est ensuite filtré afin d'éliminer les sels et le filtrat est évaporé. Le résidu est solubilisé dans 10 mL de toluène à chaud puis la solution est laissée à revenir à température ambiante. Le solide qui est cristallisé est récupéré par filtration, lavé avec 10 mL de toluène et séché sous pression réduite. On isole 2,50 g de 1,4,7-tris(*tert*-butoxycarboxyméthyle)-1,4,7,10-tétraaazacyclodécane sous la forme d'une poudre blanche. Rendement=82 %.

### Caractéristiques physico-chimiques:

**RMN¹H** (200,13 MHz, CDCl₃): δ_{H} 1,43 (18H, s, *tert*-Bu), 1,48 (9H, s, *tert*-Bu), 2,89-3,08 (16H, m, -CH₂-CH₂N-), 3,28 (2H, s, *-CH₂C0₂-),* 3,36 (4H, s, *-CH₂CO₂-),* 9,94 (1H, m, NH)

### b) Deuxième étape : Synthèse du N-{5-[4, 7,10-tris(tert-butoxycarbonylméthyl)-1,4,7,10-tétraazacyclododécan-1-yl]-méthyle}styrène de formule suivante :

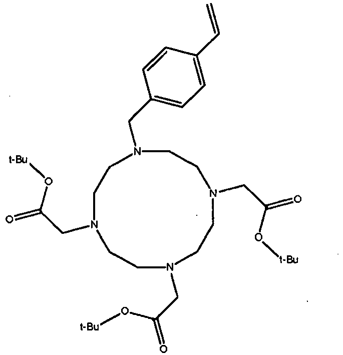

Dans un ballon bicol surmonté d'un refrigérant et sous atmosphère d'argon, on charge avec 2,40 g de 1,4,7-tris(tert-butoxycarboxyméthyle)-1,4,7,10-tétraazacyclododécane (4,67 mmoles), 1,1 g de 4-chlorovinylbenzyle (1,5 éq., 7,00 mmoles), 0,97 g de carbonate de potassium (1,5 éq., 7 mmoles), 0,7 g d'iodure de sodium (1 éq., 4,67 mmoles) et 70 mL d'acétonitrile anhydre. Le milieu réactionnel est agité et porté à la température de 70°C durant 20 heures. Le milieu est ensuite laissé revenir à température ambiante, filtré et le filtrat est évaporé sous pression réduite. L'huile obtenue est reprise dans 100 mL d'éther diéthylique et le mélange est abandonné 1 heure. Le solide, qui a précipité, est récupéré par filtration, lavé avec deux fois 30 mL d'éther diéthylique et séché sous vide. On obtient 2,46 g de N-{5-[4,7,10-Tris(*tert*-butoxycarbonylméthyl)-1,4,7,10-tétraazacyclodécan-1-yl]méthyle}styrène sous la forme d'une poudre blanche. Rendement : 84%.

### Caractéristiques physico-chimiques

**RMN¹H** (200, 13 MHz, CDCl₃): δ_{H} 1,46 (27H, s, *tert*-Bu), 2,10-2,52 (16H, m, -CH₂-CH₂N), 2,81 (6H, m, -*CH₂*CO₂-), 3,02 (2H, s, *CH₂Ar),* 5,24 (1H, d, *j* = 10,6 Hz, *CH₂=CH),* 5,70 (1H, d,j = 18 Hz, *CH₂=CH),* 6,64 (1H, dd,J = 11/18 Hz, *CH=CH₂),* 7,29 (2H, *d, j* = 9,2 Hz, CH*Ar*), 7,38 (2H, d,*j* = 9, 9 Hz, CH-*Ar*)
**RMN¹³C** (50,32 MHz, CDCl₃) : δ_{c} 27,4; 27,5; 49,4 (m.); 55,2; 55,4; 58,8; 81,9; 82,4; 113,8; 126,0; 129,8; 135,6; 136,3; 136,7; 172,0; 173,9 (2C).
**MALDI-TOF:** MH⁺ = 631,443; MNa⁺ = 653,425 (Valeurs théoriques : 631,443 et 653,425)

### c) 3^{ème} étape : Synthèse de l'acide N-{5-[4,7,10-tris(acétique)-1,4,7,10-tétraazacyclododécan-1-yl]-méthyle}-styrène (dit composé L1) de formule suivante :

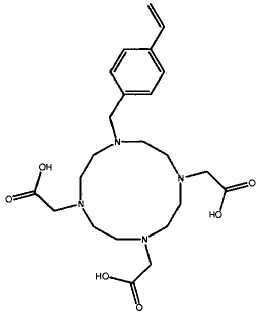

A une solution de N-{5-[4,7,10-tris(tert-butoxycarbonylméthyl)-1,4,7,10-tétraazacyclododécan-1-yl]méthyle}styrène (2,0 g, 3,17 mmoles) dans 40 mL de dichlorométhane, refroidie dans un bain de glace, on additionne goutte à goutte une solution de 10 mL d'acide trifluoroacétique dans 40 mL de dichlorométhane. Le milieu réactionnel est agité 16 heures à température ambiante puis le solvant est évaporé sous pression réduite. L'acide trifluoroacétique résiduel est co-évaporé à l'aide d'éthanol. Le résidu semi-solide obtenu est dissous dans 15 mL d'acétone anhydre et floculé dans 250 mL d'éther éthylique anhydre. Le surnageant est éliminé et, après séchage, sous vide du précipité, on isole 1,36 g d'acide N-{5-[4,7,10-tris(acétique)-1,4,7,10-tétraazacyclodécan-1-yl]-méthyle}styrène sous la forme d'une poudre jaune avec un rendement de 93%.

### Caractéristiques physico-chimiques

**RMN¹H** (200,13 MHz, DMSO-d₆) : δ_{H} 2,60-3,43 (24H, m, CH₂), 4,21 (3H, m, COO*H*), 5,26 (1H, m, *CH₂=CH), 5,79* (1H, m, *CH₂=CH),* 6,71 (1H, m, *CH=CH₂),* 7,0-7,47 (4H, m, *CH Ar)*
**RMN¹³C** (50, 32 MHz, DMSO-*d₆*) : δ_{c} 48, 6 (m); 55,8 (m); 81,2; 81,8; 115,6; 125,8; 126,6; 130,5; 135,9; 138.0; 170,0; 172,0
**Spectrométrie de masse FAB⁺** MH⁺=463 ; MNa⁺=485 (Valeurs théoriques : 463 et 485)

### EXEMPLE 4

Cet exemple illustre la synthèse d'un complexe N-{5-[4,7,10-tris(acétate)-1,4,7,10-tétraazacyclodécan-1-yl]-méthyle}styrène d'ytterbium de formule suivante : 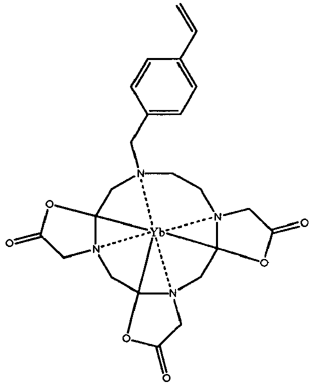 dit complexe C₁-Yb.

Une solution de L¹ (1,12g, 2,42 mmoles) dans 40 mL d'eau qualité Milli Q (Millipore) est ajustée à pH=7 à l'aide d'une solution d'hydroxyde de potassium 0,2 M. La solution est filtrée et on additionne au filtrat sous agitation et goutte à goutte une solution de chlorure d'ytterbium (0,787 g, 2 mmoles) dans 30 mL d'eau Milli Q. Le pH du milieu est ajusté à 6 en fin d'addition. Après 1H30 d'agitation à température ambiante, le pH est à nouveau ajusté à 6 puis la solution est filtrée. Le filtrat est récupéré et évaporé sous pression réduite. Le solide résiduel est repris dans 80 mL de méthanol et les sels qui précipitent sont éliminés par filtration. L'opération est répétée, jusqu'à ce qu'il ne se forme plus de précipités dans le méthanol. On isole 1,11 g de L¹-Yb sous la forme d'une poudre blanche avec un rendement de 88%.

### Caractéristiques physico-chimiques

**RMN¹H** (400,13 MHz, CD₃OD) : δ_{H} -136,0; -94,0; -89,7; - 82,4; -74,0; -71,6; -56,2; -54,9; - 33,8; -26,7; -12,6; 23,0; 24,5; 38,2; 40,4; 45,7; 65,3; 143,8; 161,9; 186,9; 210,6
**MALDI-TOF :** MH⁺ = 634,48 et MNa⁺ = 656,14 (Valeurs théoriques: 634,17 et 656,15)

### EXEMPLE 5

Cet exemple illustre la préparation du sel de formule suivante :

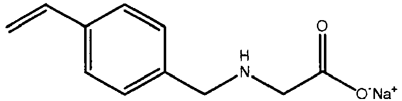

A une solution de Gly²OEt (11,3 g) dans 133 mL d'éthanol anhydre, on additionne 4,67 mL de soude à la concentration de 35% massique. Le milieu réactionnel est agité durant 16 heures à température ambiante. Le précipité formé est isolé par filtration et rincé avec 20 mL d'éthanol anhydre. Le filtrat est évaporé sous pression réduite et repris dans 30 mL d'éthanol anhydre. Le solide blanc qui précipite est récupéré par filtration. Les deux fractions sont rassemblées et après un séchage sous pression réduite, on isole 7,27 g de Gly²Na sous la forme d'une poudre blanche. Rendement : 66%.

### Caractéristiques physico-chimiques

**RMN¹H** (200,13 MHz, D₂O) : δ_{H} 3,14 (2H, s, *-CH₂CO-),* 3,69 (2H, s, *CH₂Ar),* 5,27 (1H, dd*, j* = 0,8/11,8 Hz, *CH₂=CH),* 5,82 (1H, d, *j* = 0,8/17,8 Hz, *CH₂=CH),* 6,76 (1H, dd, *j* = 10,8/17,8 Hz, CH₂=*CH*), 7,32 (2H, d*, j* = 8,2 Hz, C*HAr*), 7,47 (2H, d, *j*=8,2 Hz, C*HAr*)
**RMN¹³C** (50,32 MHz, D₂O) : δ_{c} 43,9; 51,2; 113,6; 125,8 (2C); 128,4 (2C); 135,8; 135,9; 138,0; 180,5

### EXEMPLE 6

Cet exemple illustre la préparation du sel de formule suivante :

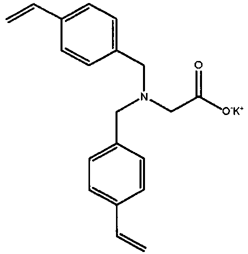

A une solution de Gly^{2'}OEt (1,00g, 2,9 mmoles) dans 10 mL d'une solution éthanol/eau distillée (90/10), on additionne 0,21 g de potasse (1,25 éq., 3,7 mmoles). Le milieu réactionnel est agité durant 5 heures à température ambiante. Les solvants sont ensuite évaporés sous pression réduite et le résidu est trituré dans 8 mL d'éther éthylique. Le précipité formé est isolé par filtration et on isole 0,48 g de Gly^{2'}K sous la forme d'une poudre blanche pure. Rendement : 47%.

### Caractéristiques physico-chimiques

**RMN¹H** (200,13 MHz, D₂O) : δ_{H} 2,96 (2H, s, *-CH₂CO-),* 3,14 (4H, s, *CH₂Ar),* 4,98 (2H, d,j = 12,0 hz, *CH₂=CH),* 5,52 (2H, *d,j* 18,0 Hz, *CH₂=CH),* 6,47 (2H, dd, *j* = 10,0/18,0 Hz, CH₂=*CH*), 7,14 (4H, *d,j =* 8,0 Hz, *CHAr),* 7,28 (4H, *d, j* = 8,0 *Hz, CHAr)*

### EXEMPLE 7

Cet exemple illustre la synthèse d'un complexe (Gly²)ₓ Yb par réaction du (Gly²)Na avec du triflate d'ytterbium.

A une solution de triflate d'ytterbium (7,05 g, 11,4 mmoles) dans 100 mL d'eau distillée, sous agitation vigoureuse, est additionné goutte à goutte Gly²Na (7,27 g, 3 éq.,34,09 mmoles) en solution dans 100 mL d'eau distillée. Après l'addition complète de Gly²Na, le pH du milieu réactionnel est ajusté à 7,5 (à l'aide de soude 1N ou d'acide chlorhydrique 1N) et porté 30 minutes à 50°C. Une fois le milieu réactionnel revenu à température ambiante, le pH est à nouveau ajusté à 7,5 et le milieu est agité pendant 90 minutes. Le précipité formé est isolé par filtration, rincé avec 20 mL d'eau distillée et séché à l'étuve durant 6 heures (40°C, sous vide). On obtient 5,46 g d'un mélange de complexes d'ytterbium sous la forme d'une poudre blanche.

### Caractéristiques physico-chimiques de la poudre blanche isolée

### Spectre MALDI-TOF: 869; 891 ; 1015 ; 1038 ; 1285 et 1658

Cette analyse MALDI-TOF (reproduite plusieurs fois) montre que la poudre est constituée d'un mélange de divers complexes d'ytterbium dinucléaires pouvant par hypothèse répondre à la formule générale suivante : **Yb₂(Gly²)ₓ(OH)_{y}(H₂O)_{z}** avec x, y et z variables.
Par exemple: si x=3, y=3 et z=4,MH⁺= 1038.
Si x=2, y=4 et z=4, MH⁺=869.

**Analyse élémentaire Yb :** % Massique observé d'Yb = 30,6% + 1,2% (valeur moyenne).

### EXEMPLE 8

Cet exemple illustre la synthèse d'un complexe formé de sels carboxylates de monomères de formule (III) et de cuivre (dit complexe (Gly^{2'})₂Cu).

A une solution de chlorure de cuivre (II) (0,18 g, 1,3 mmoles) dans 10 mL d'eau distillée, on additionne goutte à goutte le composé Gly^{2'}K (0,92 g, 2 éq., 2,7 mmoles) également en solution dans 10 mL d'eau distillée. Après l'addition complète de Gly^{2'}K, le pH du milieu réactionnel est ajusté à 10 avec une solution de soude 1N. Le milieu réactionnel est agité à température ambiante durant 3 heures. Le précipité formé est récupéré par filtration, le gâteau est lavé avec deux fois 20 mL d'eau distillée et séché à 50°C sous pression réduite durant 8 heures. On obtient 0,59 g d'une poudre de couleur violette. Rendement=65%.

### Caractéristiques physico-chimiques

**Analyse élémentaire Cu :** % Massique théorique de Cu= 9,4%±0,4%
% Massique observé de Cu = 9,5%±0,4%.

### EXEMPLE 9

Cet exemple décrit la préparation d'un matériau dopé à l'ytterbium obtenu par copolymérisation de C¹-Yb avec du divinylbenzène (DVB).

Pour ce faire, un ballon de 10 mL est chargé avec 1 mL de DMSO, 100 mg de comonomères (C¹-Yb + DVB avec des ratios variables) et 10 mg d'AiBN. La solution obtenue est dégazée par un bullage à l'argon durant 5 mn. Enfin, la solution est transférée dans un moule cylindrique en verre à l'aide d'une seringue et portée à 75°C durant 24 heures. Le gel obtenu est immergé dans l'éthanol durant une semaine afin d'échanger le DMSO par de l'éthanol. Le gel est ensuite séché au CO₂ supercritique fournissant une mousse dopée à l'ytterbium. Le taux d'ytterbium est déterminé par analyse élémentaire.

Différents essais ont été menés avec des ratios variables entre C¹-Yb/DVB et les valeurs suivantes ont été obtenues :

| | | | | |
|---|---|---|---|---|
| % Mas. théo. Yb mélange initial* | 0,9 | 3,6 | 6,3 | 13,3 |
| % Mas, obs. Yb dans mousses** | 0,56±0,04% | 2,6±0,1% | 5,1±0,2% | 12,1±0,5% |

| | | | | |
|---|---|---|---|---|
| *"% %Mas. théo. Yb"=% massique d'ytterbium dans mélange initial de monomères (avant polymérisation), **"% Mas. obs. Yb" =% massique observé d'ytterbium dans les mousses isolées. | | | | |

### EXEMPLE 10

Cet exemple illustre la préparation d'un matériau dopé à l'ytterbium par polymérisation de (Gly²)ₓYb ou copolymérisation de (Gly²)ₓYb/DVB (50/50) ou (Gly²)ₓYb/TMPTA (50/50).

Pour ce faire, un ballon de 25 mL est chargé avec 200 mg de (Gly²)ₓYb seul, de (Gly²)ₓYb/DVB (50/50) ou (Gly²)ₓYb/TMPTA (50/50), 30 mg d'AiBN, 3 mL de dibutylphtalate, 3 mL de chloroforme ou de THF et 1 mL de méthanol. Le mélange est agité à température ambiante jusqu'à l'obtention d'une solution limpide. La solution est alors placée à l'évaporateur rotatif afin d'éliminer le chloroforme et le méthanol (Température du bain = 35°C, environ 15 minutes, pression finale= 5 millibars). Après évaporation des co-solvants, on additionne alors 100 mg de co-monomère et la solution obtenue est dégazée par un bullage à l'argon durant 5 minutes. Enfin, la solution est transférée dans un moule cylindrique en verre à l'aide d'une seringue et portée à 90°C durant 16 heures.

Le gel obtenu est immergé dans l'éthanol durant une semaine afin d'échanger le dibutylphtalate par de l'éthanol. Le gel est ensuite séché au CO₂ supercritique fournissant une mousse dopée à l'ytterbium. Le taux d'ytterbium est déterminé par analyse élémentaire.

Différents essais ont été menés et les valeurs suivantes ont été obtenues :

| | (Gly²)ₓYb | (Gly²)_{X}Yb/DVB | (Gly²)ₓYb /TMPTA |
|---|---|---|---|
| % Mas. théo. Yb mélange initial * | 30,6 | 22,6 | 21,3 |
| %Mas.obs.Yb dans mousses** | 30,8± 1,2 | 22,7±0,9 | 21,4±0,9 |

| | | | |
|---|---|---|---|
| *"% %Mas. théo. Yb"=% massique d'ytterbium dans mélange initial de monomères (avant polymérisation), **"% Mas. obs. Yb" =% massique observé d'ytterbium dans les mousses isolées. | | | |

### EXEMPLE 11

Cet exemple illustre la préparation d'un matériau dopé au cuivre par polymérisation de (Gly^{2'})₂Cu.

Pour ce faire, un ballon de 10 mL est chargé avec 1 mL de DMSO, 100 mg de Gly²Cu et 10 mg d'AiBN. La solution obtenue est dégazée par un bullage à l'argon durant 5 minutes puis transférée dans un moule cylindrique en verre à l'aide d'une seringue. Le milieu réactionnel est porté à 90°C durant 24 heures. Le gel obtenu est immergé dans l'éthanol durant une semaine afin d'échanger le DMSO par de l'éthanol. Le gel est ensuite séché au CO₂ supercritique fournissant une mousse de couleur bleu dopée au cuivre. Le taux de cuivre est déterminé par analyse élémentaire.

Différents essais ont été menés et les valeurs suivantes ont été obtenues:
% massique théorique de Cu = 9,4%,
%massique observé de Cu = 7,2% ± 0,2%

## Revendications

1. Procédé de préparation d'un matériau polymérique dopé par au moins un élément métallique comprenant une étape de polymérisation d'un complexe de coordination dudit élément métallique formé dudit élément et d'un ou plusieurs ligands dudit élément, le ou lesdits ligands appartenant à au moins un monomère comprenant au moins un groupe éthylénique, ledit monomère répondant à la formule (I) : 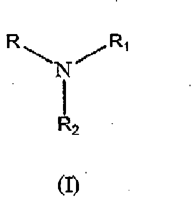 dans laquelle :
- R représente un groupe choisi parmi les groupes de formules suivantes : 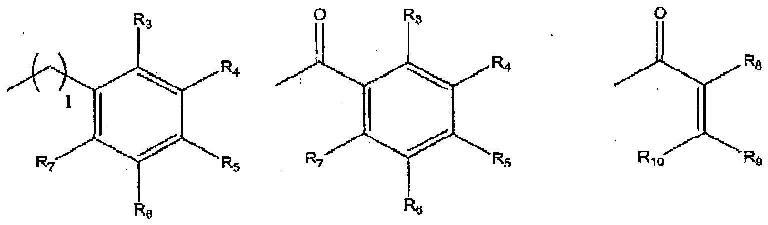
- R₁ et R₂ représentent, indépendamment, H, un groupe alkyle, un groupe aryle, un groupe de formules suivantes : 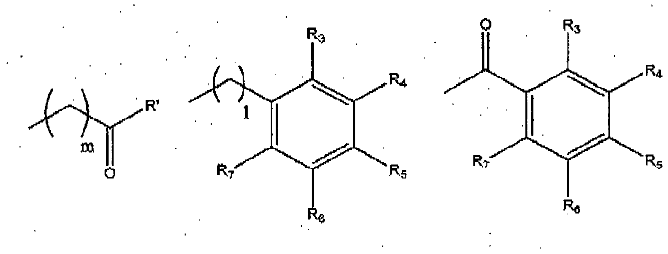 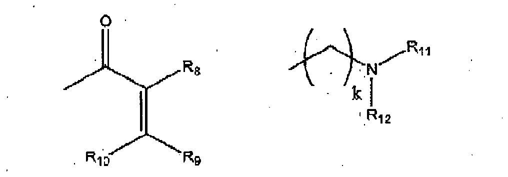 R₁₁ et R₁₂ correspondant, indépendamment, à des groupes répondant à la même définition que R₁ et R₂ donnée ci-dessus ;
- R' est un groupe OR₂₃, amine;
- R₃, R₄, R₅, R₆, R₇ représentent, indépendamment, H, un groupe éthylénique, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, - O-alkyle étant éventuellement perfluorés, un ou plusieurs atomes d'oxygène, d'azote, de soufre et/ou de sélénium pouvant s'intercaler dans lesdits groupes, à condition que l'un au moins des R₃, R₄, R₅, R₆, R₇ représente un groupe éthylénique;
- R₈, R₉ et R₁₀ représentent, indépendamment, H, un groupe éthylénique, un groupe alkyle, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, - O-alkyle étant éventuellement perfluorés, un ou plusieurs atomes d'oxygène, d'azote, de soufre et/ou de sélénium pouvant s'intercaler dans lesdits groupes ;
- R₁₃ représente H, un métal, un groupe alkyle, un groupe aryle, un groupe acyle, un groupe alkylaryle, lesdits groupes alkyle, aryle, alkylaryle étant éventuellement perfluorés et un ou plusieurs atomes d'oxygène, de soufre et/ou de sélénium pouvant s'intercaler dans lesdits groupes ;
- k, 1 et m sont des entiers allant de 0 à 20 ;
et les sels de ceux-ci,
ou répondant à la formule (IV) suivante : 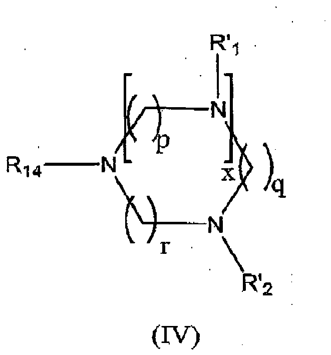 dans laquelle :
- R₁₄ représente un groupe choisi parmi les groupes de formules suivantes : 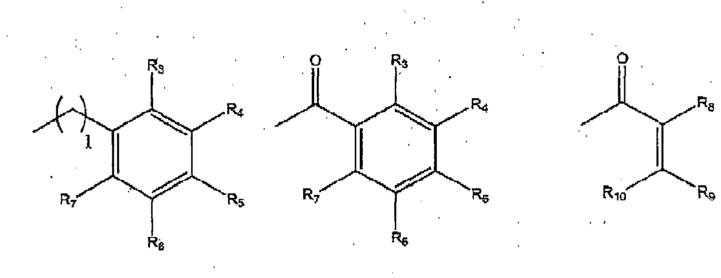 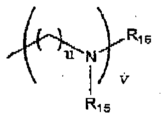
- R'₁ et R'₂ représentent, indépendamment, un groupe alkyle, un groupe aryle, un groupe de formules suivantes : 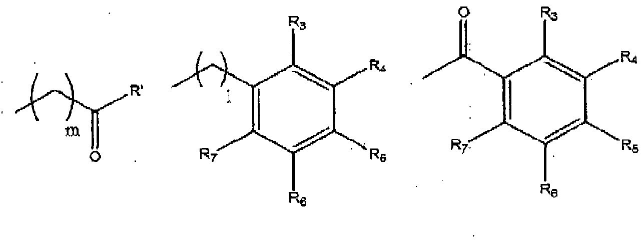 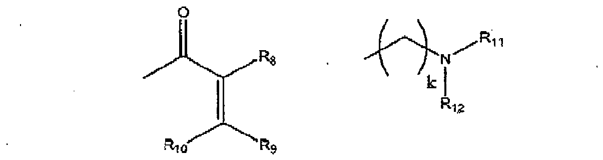 R₁₁ et R₁₂ correspondant, indépendamment, à des groupes répondant à la même définition que R'₁ et R'₂ donnée ci-dessus ;
- R' est un groupe QR₁₃, amine ;
- R₃, R₄, R₅, R₆ et R₇ représentent, indépendamment, H, un groupe éthylénique, un groupe alkyl, un groupe aryle, un groupe -O-aryle, un groupe -O-alkyle, un groupe acyle, un groupe alkylaryle, un atome d'halogène, lesdits groupes alkyle, aryle, alkylaryle, -O-aryle, -O-alkyle étant éventuellement perfluorés, un ou plusieurs atomes d'oxygène, d'azote, de soufre et/ou de sélénium pouvant s'intercaler dans lesdits groupes, à condition que l'un au moins des R₃, R₄, R₅, R₆, R₇ représente un groupe éthylénique;
- Re, R₉ et R₁₀ représentent, indépendamment, H, un groupe éthylénique, un groupe alkyle, un groupe aryle, un groupe -O-aryle, un groupe -O-alkyle, un groupe acyle, un groupe alkylaryl, un atome d'halogène, lesdits groupes alkyle, aryle, alkylaryle, -O-aryle, -O-alkyle étant éventuellement perfluorés, un ou plusieurs atomes d'oxygène, d'azote, de soufre et/ou de sélénium pouvant s'intercaler dans lesdits groupes ;
- R₁₅ représente un groupe de formules suivantes : 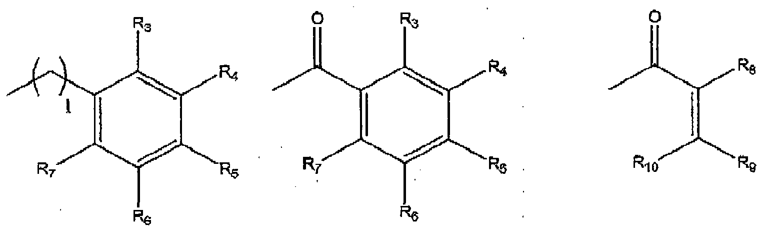 les R₃ à R_{1c} étant tels que définis ci-dessus ;
- R₁₃ représente H, un métal, un groupe alkyle, un groupe aryle, un groupe acyle, un groupe alkylaryle, lesdits groupes alkyle, aryle, alkylaryle étant éventuellement perfluorés et un ou plusieurs atomes d'oxygène, de soufre et/ou de sélénium pouvant s'intercaler dans lesdits groupes ;
- R₁₆ représente un groupe de formule : 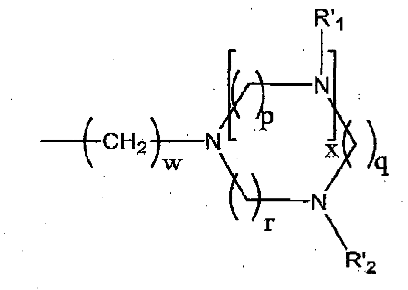 les R'₁ et R'₂ étant tels que définis ci-dessus ;
- k, l, m, u, p, q, r, x et w sont des entiers allant de 0 à 20, v est un entier allant de 1 à 20, à la condition que, lorsque x est égal à 0, (r+q) est au moins égale à 2, et lorsque x est égal à 1, au moins l'un des p, q, r est différent de 0.

2. Procédé selon à revendication 1, dans lequel R est un groupe de formule : 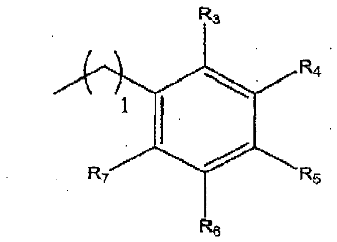 et l'un au moins des R₁ et R₂ est un groupe de formule : 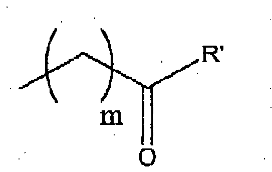 R₃ à R₇, R', l et m ayant les mêmes significations que celles exposées à la revendication 1.

3. Procédé selon la revendication 1 ou 2, dans lequel R est un groupe de formule : 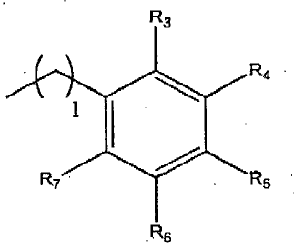 R₁ est un groupe de formule : 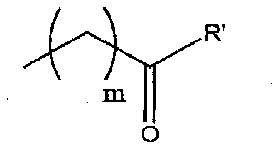 et R₂ est un atome d'hydrogène, R₃ à R₇, R', l et m ayant les mêmes significations que celles exposées à la revendication 1.

4. Procédé selon la revendication 3, dans lequel le monomère répond à la formule (II) suivante : 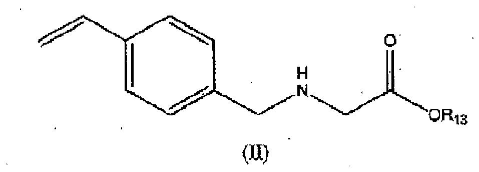 R₁₃ représentant H, un métal ou un groupe alkyle.

5. Procédé selon la revendication 1 ou 2, dans lequel R est un groupe de formule : 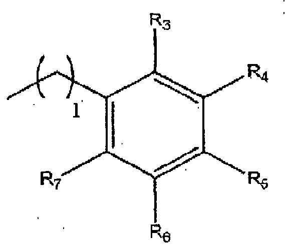 R₁ est un groupe de formule : 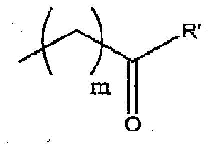 et R₂ est un groupe de formule : 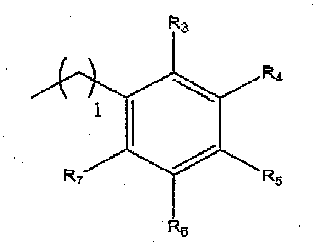 1 et m, R₃ à R₇ et R' ayant les mêmes significations que celles données à la revendication 1.

6. Procédé selon la revendication 5, dans lequel le monomère répond à la formule (III) suivante : 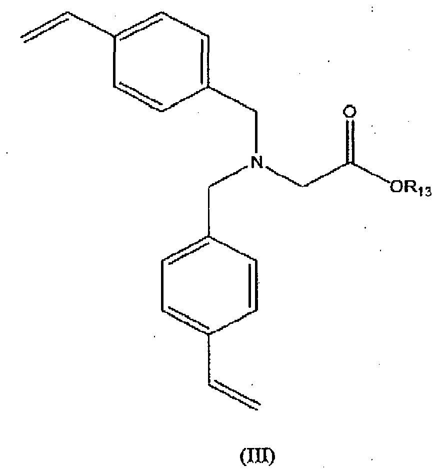 R₁₃ représentant H, un métal ou un groupe alkyle.

7. Procédé selon la revendication 1, dans lequel R₁₄ représente un groupe de formule suivante : 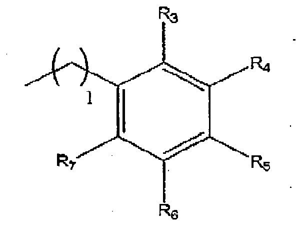 et l'un au moins des R'₁ et R'₂ représente : 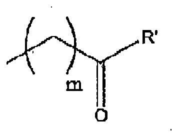 les R₃ à R₇, R', 1 et m répondant à la même définition que celle donnée à la revendication l, p, q, r et x étant au moins égaux à 1.

8. Procédé selon la revendication 1 ou 7, dans lequel R₁₄ est un groupe de formule : 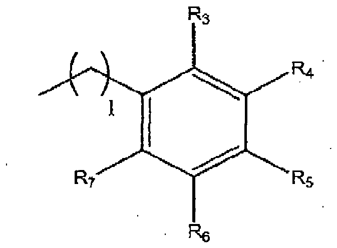 R'₁ et R'₂ représentent un groupe de formule : 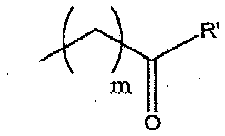 les R₃ à R₇, R', l et m répondant à la même définition que celle donnée à la revendication 1, p, q, r et x étant au moins égaux à 1.

9. Procédé selon l'une quelconque des revendications 1 et 7 ou 8, dans lequel le monomère répond à la formule (V) suivante : 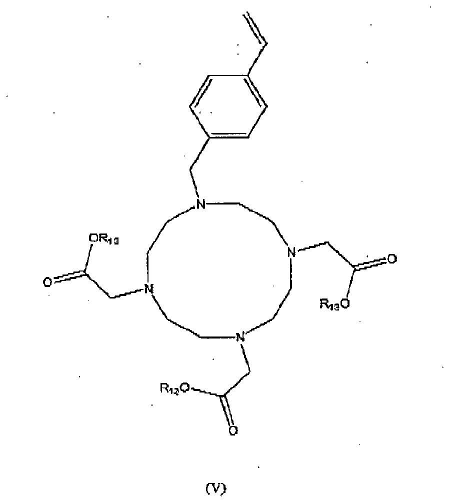 R₁₃ représentant H, un métal ou un groupe alkyle.

10. Procédé selon la revendication 1, dans lequel R₁₄ représente un groupe de formule : 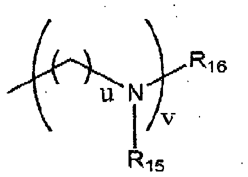 et l'un au moins des R'₁ et R'₂ représente : 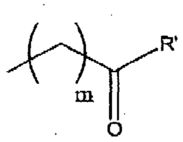 R₁₅, R₁₆, R', m, u et v répondant aux mêmes définitions que celles données à la revendication 1, p, q, r et x étant au moins égaux à 1.

11. Procédé selon la revendication 10, dans lequel R₁₅ correspond à un groupe de formule : 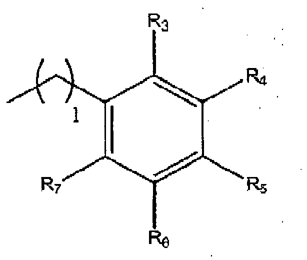 1, R₃ à R₇ répondant à la même définition que celle donnée à la revendication 1, et les groupes R'₁ et R' ₂ représentent un groupe de formule : 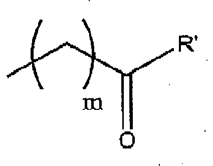 avec m et R' répondant à la même définition que celle donnée à la revendication l, p, q, r et x étant au moins égaux à 1.

12. Procédé selon la revendication 11, dans lequel le monomère répond à la formule (VI) suivante : 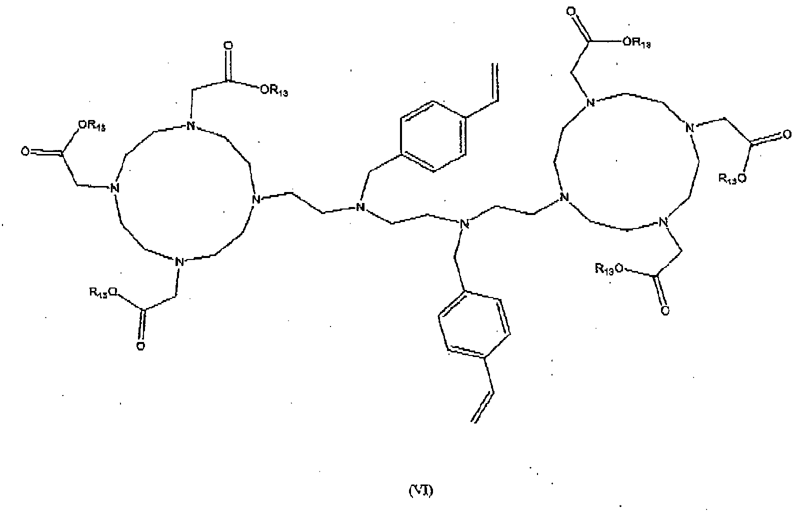 R₁₃ représentant H, un métal ou un groupe alkyle.

13. Procédé selon l'une quelconque des revendications précédentes, dans lequel l'élément métallique est choisi parmi les métaux alcalins, les métaux alcalino-terreux, les métaux de transition, les lanthanides, les actinides ainsi que les éléments Al, Ga, Ge, In, Sn, Sb, Tl, Pb, Bi ou Po.

14. Procédé selon la revendication 13, dans lequel l'élément métallique est un élément lanthanide, tel que l'ytterbium.

15. Procédé selon l'une quelconque des revendications précédentes, dans lequel l'étape de polymérisation est réalisée en présence d' un ou plusieurs comonomères différents du monomère défini selon l'une quelconque des revendications 1 à 14.

16. Procédé selon la revendication 15, dans lequel le ou les comonomères sont choisis parmi les monomères styréniques et les monomères acrylates.

17. Procédé selon la revendication 15 ou 16, dans lequel le ou les comonomères comprennent au moins deux groupes éthyléniques.

18. Procédé selon la revendication 16 ou 17, dans lequel le ou les comonomères répondent à l'une des formules (VII) ou (VIII) suivantes : 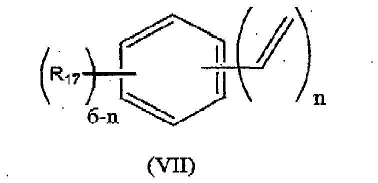 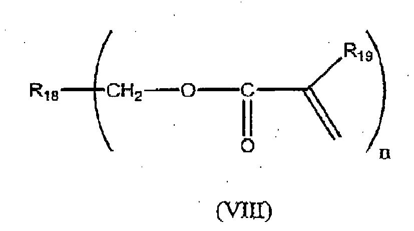 dans lesquelles les (6-n) R₁₇. identiques ou différents, représentent un atome d'hydrogène, un groupe alkyle, un groupe aryle, un groupe -O-aryle, un groupe -O-alkyle, un groupe acyle, un groupe alkylaryl, un atome d'halogène, lesdits groupes alkyle, aryle, alkylaryle, -O-aryle, -O-alkyle étant éventuellement perfluorés, R₁₈ représente un groupe alkyle, R₁₉ représente H ou un groupe alkyle et n étant un entier allant de 1 à 3.

19. Procédé selon la revendication 18, dans lequel le comonomère est le divinylbenzène.

20. Procédé selon la revendication 18, dans lequel le comonomère est le triméthylolpropanetriacrylate de formule suivante :

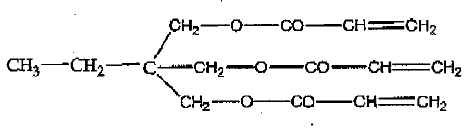

21. Procédé selon l'une quelconque des revendications précédentes, comprenant, avant l'étape de polymérisation, une étape de préparation du complexe de coordination tel que défini à la revendication 1.

22. Procédé selon l'une quelconque des revendications précédentes, comprenant, après l'étape de polymérisation, une étape de séchage supercritique au CO₂.

23. Procédé selon l'une quelconque des revendications précédentes, dans l'étape de polymérisation, consiste en :
- la copolymérisation d'un complexe de coordination formé d'un monomère de formule (V) telle que définie à la revendication 3 et d'ytterbium en présence de divinylbenzène ;
- la polymérisation d'un complexe de coordination formé de monomères de formule (II) telle que définie à la revendication 4 et d'ytterbium ;
- la copolymérisation d'un complexe de coordination formé de monomères de formule (II) telle que définie à la revendication 4 et d'ytterbium en présence de divinylbenzène ou de triméthylolpropanetriacrylate ;
- la polymérisation d'un complexe de coordination formé de monomères de formule (III) telle que définie à la revendication 6 et de cuivre.

## Patentansprüche

1. Verfahren zur Herstellung eines mit mindestens einem metallischen Element dotierten Polymermaterials, das einen Schritt der Polymerisation eines aus diesem Element und einem oder mehreren Liganden dieses Elements gebildeten Koordinationskomplexes dieses metallischen Elements umfasst, wobei der oder die Liganden wenigstens einem Monomeren angehören, das wenigstens eine Ethylengruppe umfasst und dieses Monomer der Formel (I) entspricht: 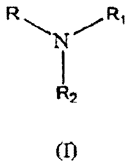 worin:
- R eine aus den folgenden Formeln ausgewählte Gruppe bedeutet: 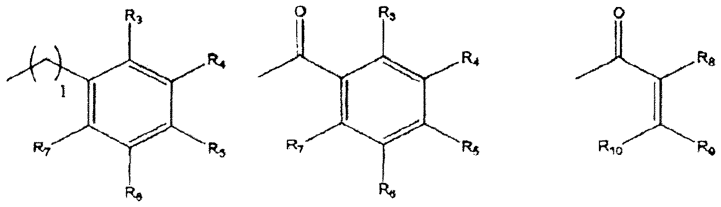
- R₁ und R₂ unabhängig H, eine Alkylgruppe, eine Arylgruppe oder eine Gruppe der folgenden Formeln bedeuten: 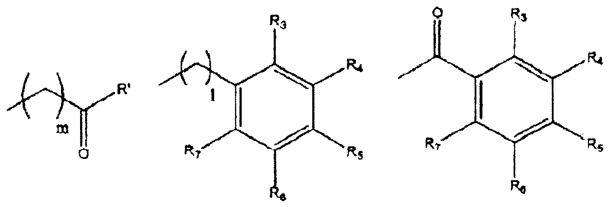 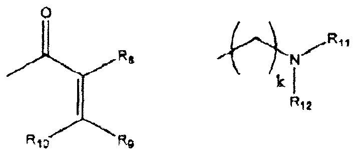 wobei R₁₁ und R₁₂ unabhängig Gruppen darstellen, die derselben, vorstehend für R₁ und R₂ angegebenen Definition entsprechen;
- R' eine Gruppe OR₁₃ oder Amin ist;
- R₃, R₄, R₅, R₆ und R₇ unabhängig H, eine Ethylengruppe, eine Alkylgruppe, eine Arylgruppe, eine Gruppe -O-Aryl, eine Gruppe -O-Alkyl, eine Acylgruppe, eine Alkylarylgruppe oder ein Halogenatom bedeuten, wobei die Alkyl-, Aryl-, Alkylaryl, -O-Aryl- und -O-Alkylgruppen gegebenenfalls perfluoriert sein können und diese Gruppen durch ein oder mehrere Sauerstoff-, Stickstoff-, Schwefel- und/oder Selenatome unterbrochen sein können, mit der Maßgabe, dass wenigstens eines von R₃, R₄, R₅, R₆ und R₇ eine Ethylengruppe bedeutet;
- R₈, Rg und M₁₀ unabhängig H, eine Ethylengruppe, eine Alkylgruppe, eine Arylgruppe, eine Gruppe -O-Aryl, eine Gruppe -O-Alkyl, eine Acylgruppe, eine Alkylarylgruppe oder ein Halogenatom bedeuten, wobei die Alkyl-, Aryl-, Alkylaryl, -O-Aryl- und -O-Alkylgruppen gegebenenfalls perfluoriert sein können und diese Gruppen durch ein oder mehrere Sauerstoff-Stickstoff-, Schwefel- und/oder Selenatome unterbrochen sein können;
- R₁₃ H, ein Metall, eine Alkylgruppe, eine Arylgruppe, eine Acylgruppe oder eine Alkylarylgruppe bedeutet, wobei die Alkyl-, Aryl- und Alkylarylgruppen gegebenenfalls perfluoriert sein können und diese Gruppen durch ein oder mehrere Sauerstoff-, Stickstoff-, Schwefel- und/oder Selenatome unterbrochen sein können;
- k, l und m ganze Zahlen von 0 bis 20 sind;
und deren Salze,
oder der folgenden Formel (IV) entspricht: 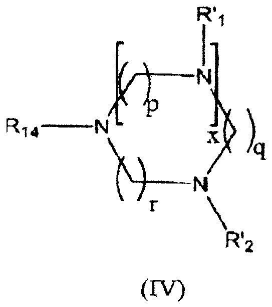 worin:
- R₁₄ eine Gruppe bedeutet, die aus den Gruppen der folgenden Formeln ausgewählt ist: 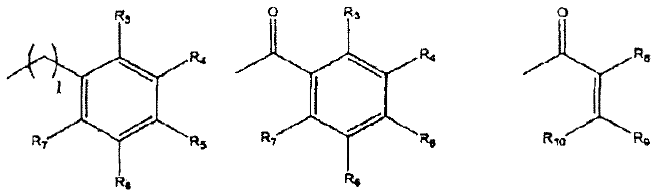 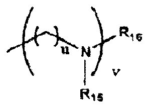
- wobei R'₁ und R'₂ unabhängig eine Alkylgruppe, eine Arylgruppe oder eine Gruppe der folgenden Formeln bedeuten: 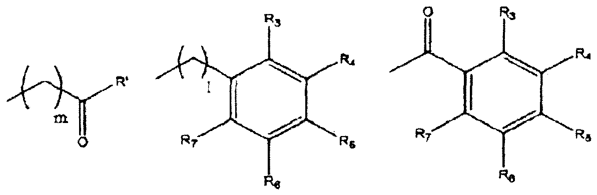 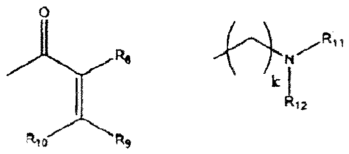 wobei R₁₁ und R₁₂ unabhängig Gruppen bedeuten, die derselben, vorstehend für R'₁ und R'₂ angegebenen Definition entsprechen;
- R' eine Gruppe OR₁₃ oder Amin ist;
- R₃, R₄, R₅, R₆ und R₇ unabhängig H, eine Ethylengruppe, eine Alkylgruppe, eine Arylgruppe, eine Gruppe -O-Aryl, eine Gruppe -O-Alkyl, eine Acylgruppe, eine Alkylarylgruppe oder ein Halogenatom bedeuten, wobei die Alkyl-, Aryl-, Alkylaryl, -O-Aryl- und -O-Alkylgruppen gegebenenfalls perfluoriert sein können und diese Gruppen durch ein oder mehrere Sauerstoff-, Stickstoff-, Schwefel- und/oder Selenatome unterbrochen sein können, mit der Maßgabe, dass wenigstens eines von R₃, R₄, R₅, R₆ und R₇ eine Ethylengruppe bedeutet;
- R₈, R₉ und R₁₀ unabhängig H, eine Ethylengruppe, eine Alkylgruppe, eine Arylgruppe, eine Gruppe -O-Aryl, eine Gruppe -O-Alkyl, eine Acylgruppe, eine Alkylarylgruppe oder ein Halogenatom bedeuten, wobei die Alkyl-, Aryl-, Alkylaryl, -O-Aryl- und -O-Alkylgruppen gegebenenfalls perfluoriert sein können und diese Gruppen durch ein oder mehrere Sauerstoff-Stickstoff-, Schwefel- und/oder Selenatome unterbrochen sein können;
- R₁₅ eine Gruppe der folgenden Formeln bedeutet: 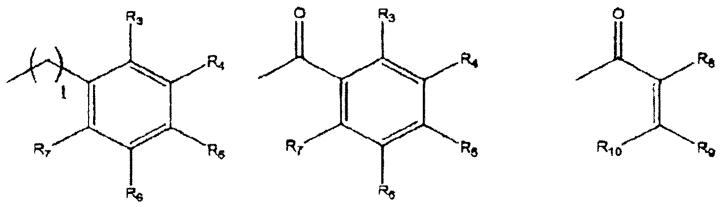 wobei R₃ bis R₁₀ wie vorstehend definiert sind;
- R₁₃ H, ein Metall, eine Alkylgruppe, eine Arylgruppe, eine Acylgruppe oder eine Alkylarylgruppe bedeutet, wobei die Alkyl-, Aryl- und Alkylarylgruppen gegebenenfalls perfluoriert sein können und diese Gruppen durch ein oder mehrere Sauerstoff-, Stickstoff-, Schwefel- und/oder Selenatome unterbrochen sein können;
- R₁₆ eine Gruppe der Formel: 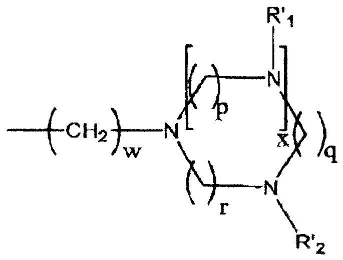 bedeutet,
worin R'₁ und R'₂ wie vorstehend definiert sind;
- k, l, m, u, p, q, r, x und w ganze Zahlen von 0 bis 20 sind, v eine ganze Zahl von 1 bis 20 ist, mit der Maßgabe, dass wenn x 0 ist, (r+q) mindestens 2 ist, und wenn x 1 ist, wenigstens eines von p, q und r von 0 verschieden ist.

2. Verfahren gemäß Anspruch 1, wobei R eine Gruppe der Formel: 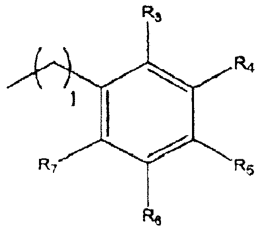 ist und wenigstens eines von R₁ und R₂ eine Gruppe der Formel 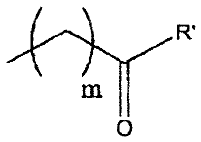 ist, wobei R₃ bis R₇, R', l und m dieselben Bedeutungen wie die in Anspruch 1 aufgeführten aufweisen.

3. Verfahren gemäß Anspruch 1 oder 2, wobei R eine Gruppe der Formel: 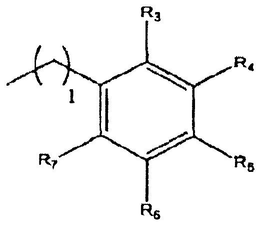 ist,
R₁ eine Gruppe der Formel 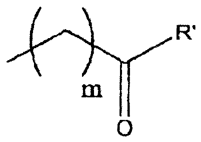 ist und R₂ ein Wasserstoffatom ist und R₃ bis R₇, R', l und m dieselben Bedeutungen wie die in Anspruch 1 aufgeführten aufweisen.

4. Verfahren gemäß Anspruch 3, wobei das Monomer der folgenden Formel (II) entspricht: 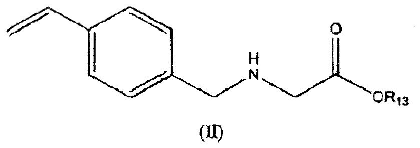 wobei R₁₃ H, ein Metall oder eine Alkylgruppe bedeutet.

5. Verfahren gemäß Anspruch 1 oder 2, wobei R eine Gruppe der Formel: 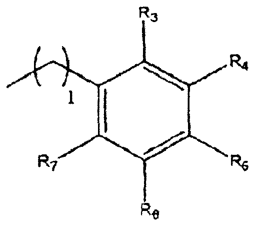 ist,
R₁ eine Gruppe der Formel: 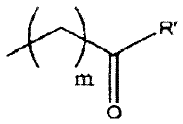 ist und R₂ eine Gruppe der Formel: 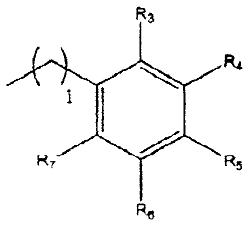 ist und
l und m, R₃ bis R₇ und R' dieselben Bedeutungen wie die in Anspruch 1 angegebenen aufweisen.

6. Verfahren gemäß Anspruch 5, wobei das Monomer der folgenden Formel (III) entspricht: 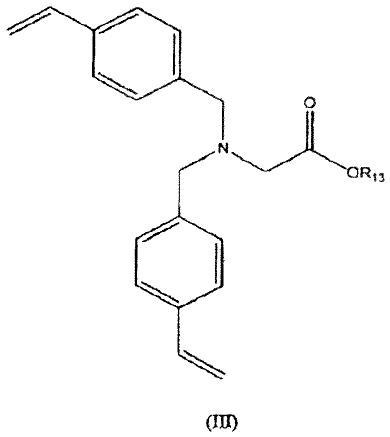 worin R₁₃ H, ein Metall oder eine Alkylgruppe bedeutet.

7. Verfahren gemäß Anspruch 1, wobei R₁₄ eine Gruppe der folgenden Formel bedeutet: 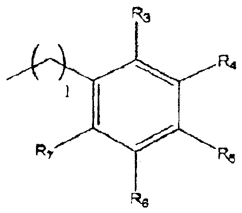 und wenigstens eines von R'₁ und R'₂ 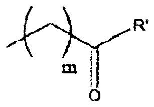 bedeutet, wobei R₃ bis R₇, R', I und m dieselben Bedeutungen wie die in Anspruch 1 aufgeführten aufweisen und p, q, r und x mindestens 1 sind.

8. Verfahren gemäß Anspruch 1 oder 7, wobei R₁₄ eine Gruppe der Formel: 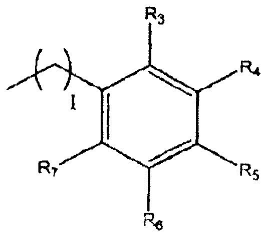 ist,
R'₁ und R'₂ eine Gruppe der Formel: 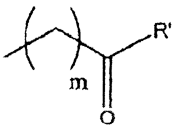 bedeuten und
R₃ bis R₇, R', l und m dieselben Bedeutungen wie die in Anspruch 1 aufgeführten aufweisen und p, q, r und x mindestens 1 sind.

9. Verfahren gemäß einem der Ansprüche 1 und 7 oder 8, wobei das Monomer der folgenden Formel (V) entspricht: 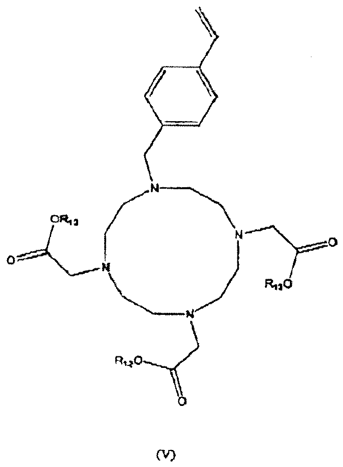 und R₁₃ H, ein Metall oder eine Alkylgruppe bedeutet.

10. Verfahren gemäß Anspruch 1, wobei R₁₄ eine Gruppe der Formel: 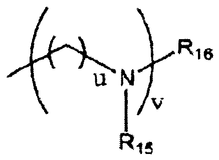 bedeutet und wenigstens eines von R'₁ und R'₂ 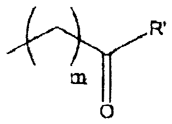 bedeutet und
R₁₅, R₁₆, R', m, u und v denselben Definitionen wie den in Anspruch 1 angegebenen entsprechen und p, q, r und x mindestens 1 sind.

11. Verfahren gemäß Anspruch 10, wobei R₁₅ einer Gruppe der Formel 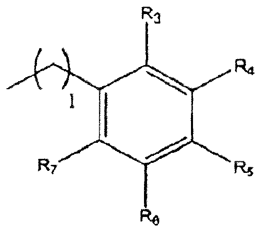 entspricht, wobei
l und R₃ bis R₇ derselben Definition wie der in Anspruch 1 angegebenen entsprechen und die Gruppen R'₁ und R'₂ eine Gruppe der Formel 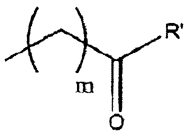 bedeuten und m und R' derselben Definition wie der in Anspruch 1 angegebenen entsprechen und p, q, r und x mindestens 1 sind.

12. Verfahren gemäß Anspruch 11, wobei das Monomer der folgenden Formel (VI) entspricht: 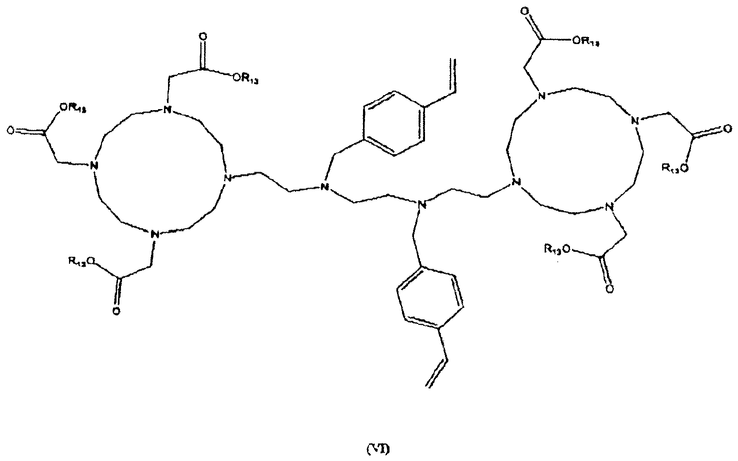 worin R₁₃ H, ein Metall oder eine Acylgruppe bedeutet.

13. Verfahren gemäß einem der vorangehenden Ansprüche, wobei das metallische Element aus Alkalimetallen, Erdalkalimetallen, Übergangsmetallen, Lanthaniden oder Aktiniden wie etwa den Elementen Al, Ga, Ge, In, Sn, Sb, TI, Pb, Bi oder Po ausgewählt ist.

14. Verfahren gemäß Anspruch 13, wobei das metallische Element ein Lanthanidenelement wie etwa Ytterbium ist.

15. Verfahren gemäß einem der vorangehenden Ansprüche, wobei der Schritt der Polymerisation in Gegenwart eines oder mehrerer Comonomeren, die von dem in einem der Ansprüche 1 bis 14 definierten Monomer verschieden sind, durchgeführt wird.

16. Verfahren gemäß Anspruch 15, wobei das oder die Comonomeren aus Styrolmonomeren oder Acrylatmonomeren ausgewählt sind.

17. Verfahren gemäß Anspruch 15 oder 16, wobei das oder die Comonomeren wenigstens zwei Ethylengruppen umfassen.

18. Verfahren gemäß Anspruch 16 oder 17, wobei das oder die Comonomeren einer der folgenden Formeln (VII) oder (VIII) entsprechen: 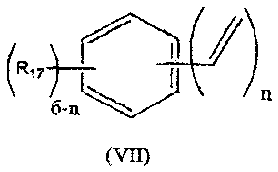 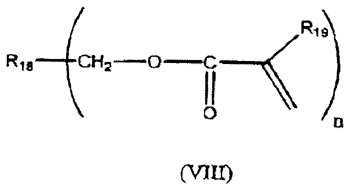 worin die (6-n) gleichen oder verschiedenen R₁₇ ein Wasserstoffatom, eine Alkylgruppe, eine Arylgruppe, eine Gruppe -O-Aryl, eine Gruppe -O-Alkyl, eine Acylgruppe, eine Alkylarylgruppe oder ein Halogenatom bedeuten und wobei diese Alkyl-, Aryl-, Alkylaryl-, -O-Aryl- und -O-Alkylgruppen gegebenenfalls perfluoriert sein können, R₁₈ eine Alkylgruppe bedeutet, R₁₉ H oder eine Alkylgruppe bedeutet und n eine ganze Zahl von 1 bis 3 ist.

19. Verfahren gemäß Anspruch 18, wobei das Comonomer Divinylbenzol ist.

20. Verfahren gemäß Anspruch 18, wobei das Comonomer Trimethylolpropantriacrylat der folgenden Formel 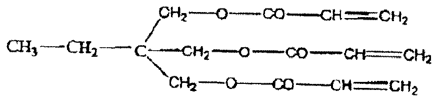 ist.

21. Verfahren gemäß einem der vorangehenden Ansprüche, das vor dem Schritt der Polymerisation einen Schritt der Herstellung eines wie in Anspruch 1 definierten Koordinationskomplexes umfasst.

22. Verfahren gemäß einem der vorangehenden Ansprüche, das nach dem Schritt der Polymerisation einen Schritt des Trocknens mit überkritischem CO₂ umfasst.

23. Verfahren gemäß einem der vorangehenden Ansprüche, wobei der Schritt der Polymerisation aus
- der Copolymerisation eines aus einem Monomeren der in Anspruch 3 definierten Formel (V) und Ytterbium gebildeten Koordinationskomplexes in Gegenwart von Divinylbenzol;
- der Polymerisation eines aus einem Monomeren der in Anspruch 4 definierten Formel (II) und Ytterbium gebildeten Koordinationskomplexes;
- der Copolymerisation eines aus einem Monomeren der in Anspruch 4 definierten Formel (II) und Ytterbium gebildeten Koordinationskomplexes in Gegenwart von Divinylbenzol oder Trimethylolpropantriacrylat;
- oder der Polymerisation eines aus einem Monomeren der in Anspruch 6 definierten Formel (III) und Kupfer gebildeten Koordinationskomplexes besteht.

## Claims

1. Process for preparing a polymeric material doped with at least one metal element, comprising a step of polymerization of a coordination complex of said metal element formed from said element and from one or more ligands of said element, said ligand(s) belonging to at least one monomer comprising at least one ethylenic group, said monomer corresponding to formula (I): 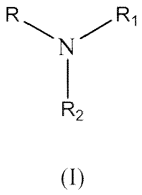 in which:
- R represents a group chosen from the groups of formulae below: 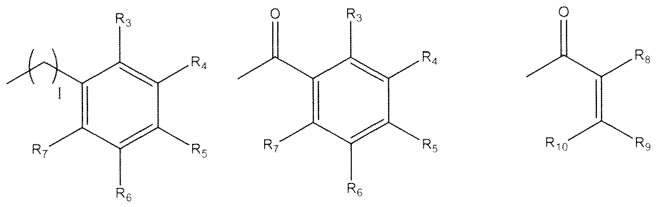
- R₁ and R₂ represent, independently, H, an alkyl group, an aryl group, a group having the formulae below: 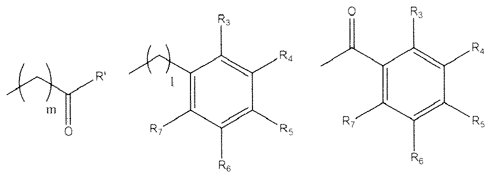 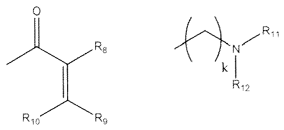 R₁₁ and R₁₂ corresponding, independently, to groups corresponding to the same definition as R₁ and R₂ given above;
- R' is an OR₁₃ or amine group;
- R₃ , R₄, R₅, R₆ and R₇ represent, independently, H, an ethylenic group, an alkyl group, an aryl group, an -O-aryl group, an -O-alkyl group, an acyl group, an alkylaryl group, a halogen atom, said alkyl, aryl, alkylaryl, -O-aryle and -O-alkyl groups being optionally perfluorinated, it being possible for one or more oxygen, nitrogen, sulphur and/or selenium atoms to be intercalated into said groups, with the proviso that at least one of R₃, R₄, R₅, R₆ and R₇ represents an ethylenic group;
- R₈, R₉ and R₁₀ represent, independently, H, an ethylenic group, an alkyl group, an aryl group, an -O-aryl group, an -O-alkyl group, an acyl group, an alkylaryl group, a halogen atom, said alkyl, aryl, alkylaryl, -O-aryl and -O-alkyl groups being optionally perfluorinated, it being possible for one or more oxygen, nitrogen, sulphur and/or selenium atoms to be intercalated in said groups;
- R₁₃ represents H, a metal, an alkyl group, an aryl group, an acyl group or an alkylaryl group, said alkyl, aryl and alkylaryl groups being optionally perfluorinated, and it being possible for one or more oxygen, sulphur and/or selenium atoms to be intercalated in said groups;
- k, 1 and m are integers ranging from 0 to 20;
and the salts thereof,
or corresponding to formula (IV) below: 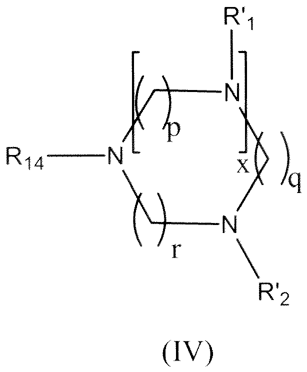 in which:
- R₁₄ represents a group chosen from the groups of formulae below: 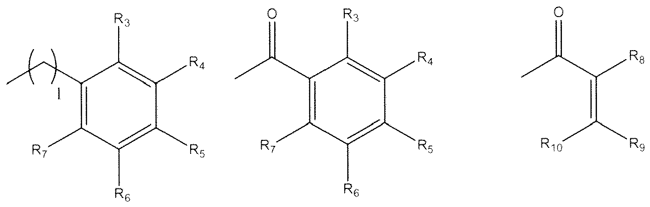 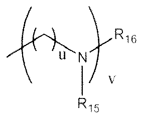
- R'₁ and R'₂ represent, independently, an alkyl group, an aryl group, or a group having the formulae below: 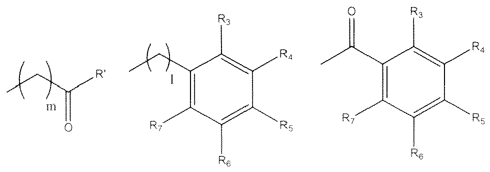 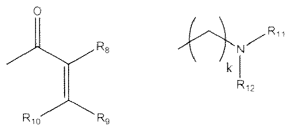 R₁₁ and R₁₂ corresponding, independently, to groups corresponding to the same definition as R'₁ and R'₂ given above;
- R' is an OR₁₃ or amine group;
- R₃, R₄, R₅, R₆ and R₇ represent, independently, H, an ethylenic group, an alkyl group, an aryl group, an
- O-aryl group, an -O-alkyl group, an acyl group, an alkylaryl group, a halogen atom, said alkyl, aryl, alkylaryl, -O-aryle and -O-alkyle groups being optionally perfluorinated, it being possible for one or more oxygen, nitrogen, sulphur and/or selenium atoms to be intercalated in said groups, with the proviso that at least one of R₃, R₄, R₅, R₆ and R₇ represents an ethylenic group;
- R₈, Rg and R₁₀ represent, independently, H, an ethylenic group, an alkyl group, an aryl group, an -O-aryl group, an -O-alkyl group, an acyl group, an alkylaryl group, a halogen atom, said alkyl, aryl, alkylaryl, -O-aryl and -O-alkyl groups being optionally perfluorinated, it being possible for one or more oxygen, nitrogen, sulphur and/or selenium atoms to be intercalated in said groups;
- R₁₅ represents a group having the formulae below: 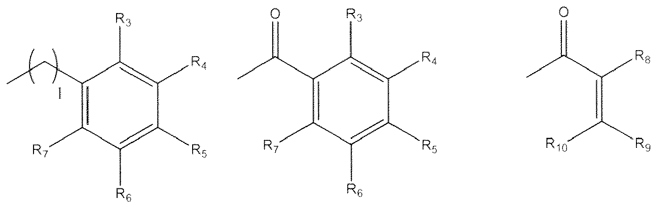 the R₃ to R₁₀ being as defined above;
- R₁₃ represents H, a metal, an alkyl group, an aryl group, an acyl group, an alkylaryl group, said alkyl, aryl and alkylaryl groups being optionally perfluorinated, and it being possible for one or more oxygen, sulphur and/or selenium atoms to be intercalated in said groups;
- R₁₆ represents a group of formula: 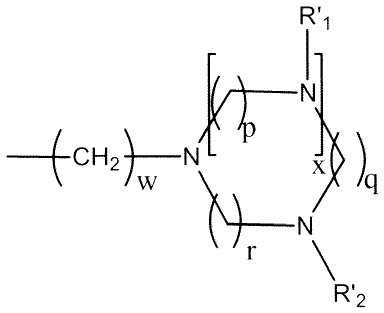 the R'₁ and R'₂ being as defined above;
- k, l, m, u, p, q, r, x and w are integers ranging from 0 to 20, v is an integer ranging from 1 to 20, with the proviso that, when x is equal to 0, (r+q) is at least equal to 2, and when x is equal to 1, at least one of p, q and r is other than 0.

2. Process according to Claim 1, in which R is a group of formula: 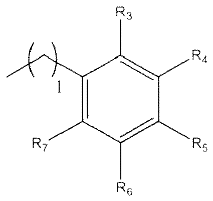 and at least one of R₁ and R₂ is a group of formula: 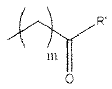 R₃ to R₇, R' , l and m having the same meanings as those disclosed in Claim 1.

3. Process according to Claim 1 or 2, in
which R is a group of formula: 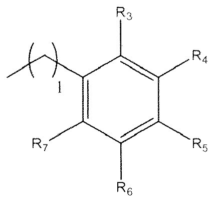 R₁ is a group of formula: 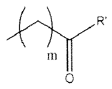 and R₂ is a hydrogen atom, R₃ to R₇, R' , l and m having the same meanings as those disclosed in Claim 1.

4. Process according to Claim 3, in which the monomer corresponds to formula (II) below: 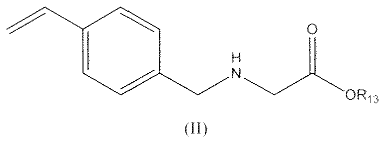 R₁₃ representing H, a metal or an alkyl group.

5. Process according to Claim 1 or 2, in which R is a group of formula: 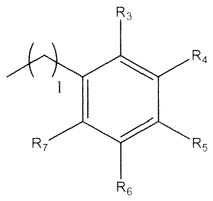 R₁ is a group of formula: 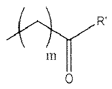 and R₂ is a group of formula: 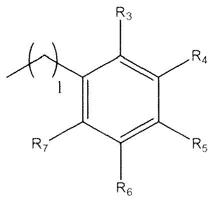 1 and m, R₃ to R₇ and R' having the same meanings as those given in Claim 1.

6. Process according to Claim 5, in which the monomer corresponds to formula (III) below: 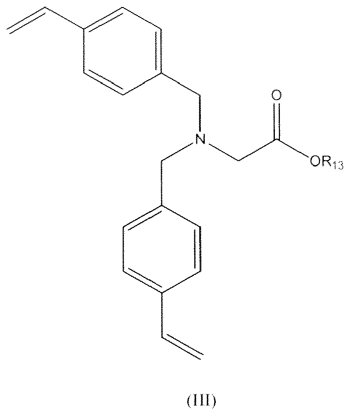 R₁₃ representing H, a metal or an alkyl group.

7. Process according to Claim 1, in which R₁₄ represents a group of formula below: 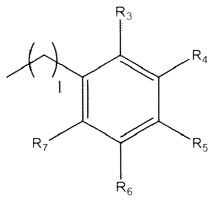 and at least one R' and R'₂ represents: 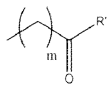 the R₃ to R₇, R', 1 and m corresponding to the same definition as that given in Claim 1, p, q, r and x being at least equal to 1.

8. Process according to Claim 1 or 7, in which R₁₄ is a group of formula: 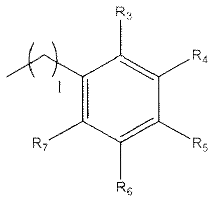 R'₁ and R'₂ represent a group of formula: 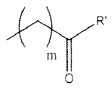 the R₃ to R₇, R', 1 and m corresponding to the same definition as that given in Claim 1, p, q, r and x being at least equal to 1.

9. Process according to any one of Claims 1 and 7 or 8, in which the monomer corresponds to formula (V) below: 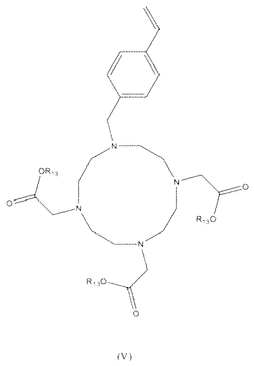 R₁₃ representing H, a metal or an alkyl group.

10. Process according to Claim 1, in which R₁₄ represents a group of formula: 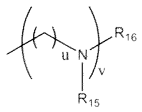 and at least one of R' and R'₂ represents: 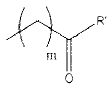 R₁₅, R₁₆, R' , m, u and v corresponding to the same definitions as those given in Claim 1, p, q, r and x being at least equal to 1.

11. Process according to Claim 10, in which R₁₅ corresponds to a group of formula: 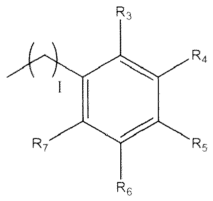 l, and R₃ to R₇ corresponding to the same definition as that given in Claim 1, and the groups R'₁ and R'₂ represent a group of formula: 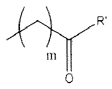 with m and R' corresponding to the same definition as that given in Claim 1, p, q, r and x being at least equal to 1.

12. Process according to Claim 11, in which the monomer corresponds to formula (VI) below: 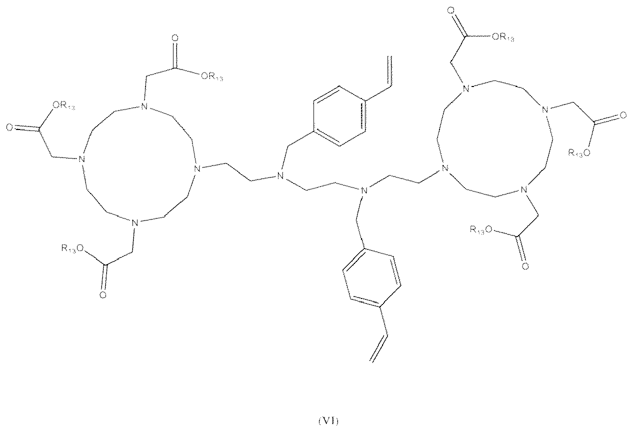 R₁₃ representing H, a metal or an alkyl group.

13. Process according to any one of the preceding claims, in which the metal element is chosen from alkali metals, alkaline-earth metals, transition metals, lanthanides, actinides, and also the elements Al, Ga, Ge, In, Sn, Sb, Tl, Pb, Bi or Po.

14. Process according to Claim 13, in which the metal element is a lanthanide element, such as ytterbium.

15. Process according to any one of the preceding claims, in which the polymerization step is carried out in the presence of one or more comonomers different from the monomer defined according to any one of Claims 1 to 14.

16. Process according to Claim 15, in which the comonomer(s) is (are) chosen from styrene monomers and acrylate monomers.

17. Process according to Claim 15 or 16, in which the comonomer(s) comprise(s) at least two ethylenic groups.

18. Process according to Claim 16 or 17, in which the comonomer(s) correspond(s) to either of formulae (VII) and (VIII) below: 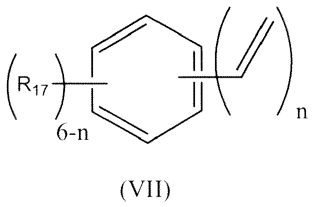 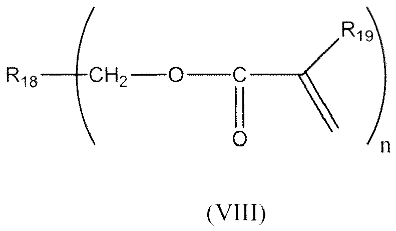 in which the (6-n) R₁₇ which may be identical or different, represent a hydrogen atom, an alkyl group, an aryl group, an -O-aryl group, an -O-alkyl group, an acyl group, an alkylaryl group, a halogen atom, said alkyl, aryl, alkylaryl, -O-aryl and -O-alkyl groups being optionally perfluorinated, R₁₈ represents an alkyl group, R₁₉ represents H or an alkyl group, and n being an integer ranging from 1 to 3.

19. Process according to Claim 18, in which the comonomer is divinylbenzene.

20. Process according to Claim 18, in which the comonomer is trimethylolpropanetriacrylate of formula below:

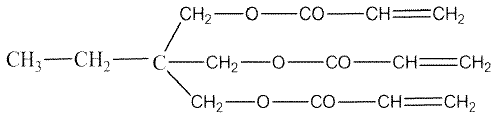

21. Process according to any one of the preceding claims, comprising, before the polymerization step, a step of preparing the coordination complex as defined in Claim 1.

22. Process according to any one of the preceding claims, comprising, after the polymerization step, a supercritical CO₂ drying step.

23. Process according to any one of the preceding claims, in which the polymerization step consists of:
- the copolymerization of a coordination complex formed from a monomer of formula (V) as defined in Claim 9 and from ytterbium, in the presence of divinylbenzene;
- the polymerization of a coordination complex formed from monomers of formula (II) as defined in Claim 4 and from ytterbium;
- the copolymerization of a coordination complex formed from monomers of formula (II) as defined in Claim 4 and from ytterbium, in the presence of divinylbenzene or of trimethylolpropanetriacrylate;
- the polymerization of a coordination complex formed from monomers of formula (III) as defined in Claim 6 and from copper.
